(19) Europäisches Patentamt
European Patent Office
Office européen des brevets

(11) **EP 2 051 074 A1**

(12) **EUROPEAN PATENT APPLICATION**

(43) Date of publication:
**22.04.2009 Bulletin 2009/17**

(51) Int Cl.:
*G01N 33/53* (2006.01)    *G01N 33/543* (2006.01)
*G01N 33/557* (2006.01)    *G01N 1/14* (2006.01)

(21) Application number: **09152328.2**

(22) Date of filing: **05.09.2002**

(84) Designated Contracting States:
**AT BE BG CH CY CZ DE DK EE ES FI FR GB GR IE IT LI LU MC NL PT SE SK TR**

(30) Priority: **05.09.2001 US 948058**

(62) Document number(s) of the earlier application(s) in accordance with Art. 76 EPC:
**02801194.8 / 1 438 585**

(71) Applicant: **Invitrogen Corporation
Carlsbad, California 92008 (US)**

(72) Inventors:
• **Bushway, Paul
San Diego, CA 92057 (US)**
• **Warden, Laurence
Poway, CA 92064 (US)**

• **Peterson, Todd
Coronado, CA 92118 (US)**
• **Kohne, David E.
La Jolla, CA 92037 (US)**
• **Yguerabide, Juan
La Jolla, CA 92037 (US)**

(74) Representative: **Williams, Richard Andrew Norman
Harrison Goddard Foote
40- 43 Chancery Lane
London
WC2A 1JA (GB)**

Remarks:
This application was filed on 06-02-2009 as a divisional application to the application mentioned under INID code 62.

(54) **Sample device preservation**

(57)    A method for preserving a sample is described. A method for preserving a sample device such as microarrays, slides and membranes is described. The preservation is achieved by applying a coating composition to a sample or sample device, and curing the coating composition. Candidate coating materials for forming the coating compositions are described. Preferably, the coating composition is an optically clear, solidifying solution. Also described are preservation kits which provide materials and instructions for the preservation of sample devices.

Calibration devices are also described.

Figure 1A

**Figure 1B**

**Figure 1C**

**Description**

This application is a continuation-in-part of U.S. Serial No. 09/948,058, filed on September 5, 2001, which is incorporated herein by reference in its entirety.

## 1. <u>INTRODUCTION</u>

The present invention relates to the field of analyte assays using detectable labels, with particular application to preservation of samples labeled with light scattering particle labels.

## 2. <u>BACKGROUND OF THE INVENTION</u>

With the recent advances in biochemistry and molecular genetics, much knowledge and information have been accumulated regarding the human body and its processes. There is a great urgency to translate this knowledge into healthcare applications and to further our understanding of various diseases. Thus, there is a great need for quantitative, multi-analyte, and inexpensive procedures and instruments for the detection of analytes. Such procedures, test kits, and instruments would be useful in research, individual point of care situations (doctor's office, emergency room, out in the field, etc.), and in high throughput testing applications.

The use of chromogenic labels, radioactive labels, chemiluminescent labels, fluorescent labels, light absorbing labels, and light scattering labels in analyte assays is well known. In particular, the recent developments in the use of resonance light scattering (RLS) particle labels and signal detection technologies have enabled a whole range of analytical applications ranging from single analyte assays, multiple analyte assays to in situ labeling of histological sections and cells.

Such RLS particle labels and their use, especially in analyte assays, are described in Yguerabide et al., U.S. Patent 6,214,560, PCT/US/97/06584 (WO 97/40181 and Yguerabide et al., PCT/US98/23160 (WO 99/20789), U. S. Patent Application Serial No. 08/953,713, by Yguerabide et al., entitled "Analyte Assay Using Particulate Labels," filed October 17, 1997, and U.S. Patent Application Serial No. 10/084,844, by Yguerabide et al., entitled "Methods For Providing Extended Dynamic Range in Analyte Assays," filed February 25, 2002, all of which are incorporated herein by reference in their entireties, including drawings. Elements of the technology are also described in two related articles by Yguerabide & Yguerabide, (1998) Anal. Biochem. 261:157-176; and (1998) Anal. Biochem. 262:137-156, which are likewise incorporated herein by reference in their entireties.

Similar methods utilizing light scattering (referred to as "plasmon resonance") labels in assays are also described in Schultz, et al, PCT/US98/02995 (WO 98/37417), Schultz et al., U.S. Patent 6,180,415, Schultz et al., U.S. Patent Application Serial No. 09/740,615 and Schultz et al., Proc. Natl. Acad. Sci., 97:995-1001 (2000).

Swope et al., U.S. Patent 5,350,697 describes apparatus to measure scattered light by having the light source located to direct light at less than the critical angle toward the sample. The detector is located to detect scattered light outside the envelope of the critical angle.

De Mey et al., U.S. Patent 4,446,238, describes a similar bright field light microscopic immunocytochemical method for localization of colloidal gold labeled immunoglobulins as a red colored marker in histological sections. The method of Immuno Gold Staining (IGS) based on optical absorbance properties of the particle labels as described by the authors:

> "In both procedures the end-product is an accumulation of large numbers of gold granules over antigen-containing areas, thus yielding the typical reddish colour of colloidal gold sols."

DeBrabander et al., U.S. Patent 4,752,567 describes a method for detecting individual metal particles of a diameter smaller than 200nm by use of bright field or epi-polarization microscopy and contrast enhancement with a video camera is described. The inventors state:

> "Typically, in the above mentioned procedures, the employed metal particles have a diameter of from about 10 to about 100nm. This is well below the resolution limit of bright field microscopy, which is generally accepted to lie around 200 nm. It is therefore quite logical that all previously known visual light microscopic methods are limited in their applications to the detection of immobilized aggregates of metal particles. Individual particles could be observed with ultramicroscopic techniques only, in particular with electron microscopy.
> It has now quite surprisingly been found that individual metal particles of a diameter smaller than 200 nm can be made clearly visible by means of bright field light microscopy or epi-polarization microscopy in the visible spectrum, provided that the resulting image is subjected to electronic contrast enhancement."

DeBrabander et al.,(1986) Cell Motility and the Cytoskeleton 6:105-113, (and U.S. Patent 4,752,567) describe use of submicroscopic gold particles and bright field video contrast enhancement. Specifically, the cells were observed by bright field video enhanced contrast microscopy with gold particles of 5-40 nanometers diameters. The authors described use of epi-illumination with polarized light and collection of reflected light or by use of a "easier and apparently more sensitive way" with a transmitted bright field illumination using monochromatic light and a simple camera.

In the Yguerabide methods of using RLS particle labels (see, for example, U.S. Patent 6,214,560), the detection and/or measurement of the light-scattering properties of the particle is correlated to the presence, and/or amount, or absence of one or more analytes in a sample. Such methods include detection of one or more analytes in a sample by binding those analyte to at least a population of detectable light scattering particle, with a size preferably smaller than the wavelength of the illumination light. The particles are illuminated with a light beam under conditions where the light scattered from the beam by the particle can be detected by the human eye with less than 500 times magnification. The light that is scattered from the particle is then detected under those conditions as a measure of the presence of those one or more analytes. By simply ensuring appropriate illumination and ensuring maximal detection of specific scattered light, an extremely sensitive method of detection can result.

Commonly, for labeled samples on a solid phase or membrane sample device, the sample is best handled with care to avoid surface damage or other degradation. This is particularly the case where it is desired to postpone reading of signal from the device until some later time or to repeat reading at a later time or to provide a permanent physical record of an experimental result. However, many types of labels are not amenable to repeated readings and/or postponed readings due to changes in the label itself. For example, fluorescent labels are subject to bleaching and fading, limiting or eliminating the ability to obtain reproducible repeat readings or reliable delayed readings. Likewise, commonly used radiolabels have relatively short half-lives, limiting the ability to delay reading of labeled samples. In contrast, resonance light scattering (RLS) particle labels, particularly metal particle light scattering labels, are not subject to such degradation, and can be reproducibly subjected to repeated readings and can provide reliable and accurate delayed readings.

The disclosed technique is broadly applicable to most sample types, systems, and assay formats as a signal generation and detection system for analyte detection. However, the assays are at times susceptible to contamination dust, e.g., on the substrate, which also scatter light and can result in increased scattering background, and artifacts in imaging. There is thus a need for methods of preserving samples and assays, which can greatly reduces the background light scattering associated with such contamination or other light scattering artifacts.

Samples of various types have been preserved in a variety of ways. For example, stained tissue samples on microscope slides have been coated or embedded in a clear material. Such preserved samples have commonly been used for classroom use to allow a number of different individuals to utilize the sample over a period of time. However, such samples are not generally used to provide quantitative results, but rather are used for qualitative microscopic inspection and teaching.

Likewise, in electron microscopy, it is common to embed a sample in a solid matrix prior to sectioning and inspection. In yet another example, agarose or polyacrylamide gels containing stained sample are often dried to provide a semi-permanent record of electrophoresis results. However, such drying typically introduces significant distortions as the gel dimensions change during the drying process.

In many circumstances involving detectable labels that specifically associate with a particular material, it is useful to be able to preserve the labeled sample. For example, in many situations, it is beneficial to be able to compare results for samples assayed at different times. However, the ability to carry out such comparisons have been limited because of instabilities of the sample, instabilities of the assay apparatus, and/or instabilities of the sample device (sample carrier). Likewise, it is often beneficial to be able to carry out repeated detection of signal from a sample device for a variety of other reasons, or to carry out detection of signal after some extended period of time instead of essentially immediately or to establish a permanent physical record of an experimental or clinical assay result. For these applications also, the ability to perform repeat or delayed detection has been limited by the various instabilities.

Thus, it would be highly advantageous to have a method and materials that would assist in preserving, protecting, and/or enhancing detection for labeled samples.

## 3. SUMMARY OF THE INVENTION

The invention provides methods for preserving a sample comprising light scattering particles or having been contacted with light scattering particles. The methods comprise applying a coating composition to at least a portion of a sample to form an optically transmissive coating. The light scattering particles are preferably between 1 and 500 nm in size, inclusive, and possess light scattering properties such that the light scattered from the light scattering particles can be detected by a human eye with less than 500 times magnification and without electronic amplification. In one embodiment, the invention provides methods for preserving a sample comprising scattered light detectable particles such that the sample can be used repeatedly and stored for extended periods of time. The coating composition used to preserve a sample can comprise a lacquer, a varnish or a wood finishing lacquer. In a specific embodiment, the coating composition comprises a polymeric compound such as alkyd resins, acrylics, carbohydrate polymers, epoxy resins, polyesters, polyurethanes, polyvinyl alcohols, polyvinyl acetates, terpenes, urethane alkyds, and urethane oils, and a diluent such as 2-butanone, 2-butoxyethanol, methyl ethyl ketone, ethylene glycol monobutyl ether, toluene or xylene. In a specific embodiment, the sample is present on a membrane, and the coating composition simultaneously modifies the membrane such that less light is scattered by the membrane and preserves the membrane for postponed and delayed analysis.

In another embodiment, the invention provides a sample device comprising at least one optically transmissive coating that is formed on a sample that comprises light scattering particles or that has been contacted with light scattering particles. The light scattering particles are preferably of a size between 1 and 500 nm inclusive, and possess light scattering properties such that the light scattered from the particles can be detected by a human eye with less than 500 times magnification and without electronic amplification.

In yet another embodiment, the invention provides a kit comprising a coating composition, and a set of instructions for coating a sample. Other components in the kit may include light scattering particles, curing agents, removal agents, sample devices, and an instrument for detection.

In yet another embodiment, the invention provides a method for reducing background light scattering or enhancing specific detection of light scattering particle labels in a sample comprising light scattering particle or having been contacted with light scattering particles. The method comprises coating at least a portion of said sample with a coating composition, where the coating composition forms an optically transmissive coating, and where the refractive index of the optically transmissive coating provides reduced background light scattering or refractive index enhancement for detection of light scattered from said labels.

While the coating of the sample device can be subjected to photo-damage, generally such damage will be negligible. Preferably the coated samples are stored under dark conditions which include measures to reduce UV exposure as much as possible. Conventional methods for dark storage conditions can be used, e.g., use of light blocking containers or storage in a dark room.

In yet another embodiment, the invention provides a method for preparing a calibration device, comprising depositing a known amount of light scattering particles at one or more discrete locations on a sample device, and coating the sample device with a coating composition that forms an optically transmissive coating. The light scattering particles are preferably of a size between 1 and 500 nm inclusive, and possess light scattering properties such that the light scattered from the particles can be detected by a human eye with less than 500 times magnification and without electronic amplification. In a specific embodiment, the invention also provides a calibration device comprising at least one discrete location that comprises a known amount of the light scattering particles and that is preserved permanently with an optically transmissive coating.

To use the calibration device, the invention also provides a method for analyzing light signals generated by a set of light scattering particles. The method comprises measuring the scattered light signals from a set of light scattering particles under defined conditions, measuring the scattered light signals from a known amount of light scattering particles under the same defined conditions, and comparing the scattered light signals from the two sets of measurements to provide an estimate of the amount of light scattering particles in the first set of particles, where the known amount of light scattering particles present on a calibration device is preserved permanently with an optically transmissive coating.

## 4. BRIEF DESCRIPTION OF THE DRAWINGS

Figures 1A, B and C illustrate the real and imaginary parts of the refractive index of gold, silver and selenium, respectively.

Figure 2 illustrates the relative scattering cross-section vs. wavelength in nanometers for various metals.

Figures 3A and 3B illustrate the normalized scattering cross-section vs. wavelength (of incident light in nanometers) for silver particles of size 20 - 100 nm, and 100-140 nm.

Figures 4A and 4B illustrate the normalized scattering cross-section vs. wavelength (of incident light in nanometers) for gold particles of size 20 - 140 nm, and 160-300 nm.

Figures 5A, B, and C show diagrams of MLSP (Manipulatable Light Scattering Particle) mixed composition particles. In Figure 8A, (1) is a core magnetic or ferroelectric material coated with (2) the desired light scattering material; Figure 8B shows (4) a light scattering material core coated with (3) magnetic or ferroelectric material; Figure 8C shows a mixture of (5) light scattering material with (6) magnetic or ferroelectric material.

Figures 6A, B, and C show dimer, tetramer, and higher order particle constructs respectively for orientable MLSP particles. Particles (1) are light scattering detectable particles and (2) are magnetic or ferroelectric particles. The line (3) is the linkage chemical, ionic, or other that binds the particles together in the multi-particle construct.

Figure 7 illustrates the particle type configurations considered when selecting particles with the desired light scattering properties.

Figure 8 is a bar graph showing exemplary signal to background ratios for several coating materials on glass slides with 80 nm gold RLS particles.

Figure 9 is a microarray layout used for illustrating the membrane transparifying and preserving method.

Figure 10 is a bar graph showing exemplary signal to background ratios for 3 lacquer solutions used as coating materials on nitrocellulose membrane with 80 nm gold RLS particles. The identifier, d100 refers to 100% Deft™ lacquer. D50egme50 refers to a solution of 50% Deft lacquer and 50% 2-butoxyethanol. P50egme50 refers to a solution of 50% Parks lacquer and 50% 2-butoxyethanol.

## 5. DETAILED DESCRIPTION OF THE INVENTION

The present invention relates to compositions and devices useful in analyte detection methods that are based on scattered light detectable particles.

In one embodiment, the invention relates to compositions of matter, formulations, and processes useful for preserving a sample which contains one or more labels of interest. The present invention can be applied to any sample device for which it is desired to immobilize and protect detectable labels, especially photodetectable labels. In a preferred embodiment, the compositions and methods of the invention are applied to immobilize and protect labels used in an analyte assay.

While the use of light scattering particles as labels overcame disadvantages of other types of labels, such as fading, it shares the common problem for any labeled samples present on a solid phase or membrane sample device. The sample must be handled with care to avoid damage if it is to be preserved or read more than once. The inventors also observed that assays based on light scattering particle labels can at times be susceptible to problems when reading is postponed or on storage, for example, presence of particulate dust or dirt on the sample which also scatter light and can result in increased scattering background and artifacts in imaging.

With this problem of robustness in mind, the inventors discovered the present methods of preserving samples and assays, which can greatly improve data quality and reduce the risk of erroneous results. Thus, in a first aspect, the invention provides a method for preserving a sample with light scattering particles comprising coating or covering the light scattering particles with a protective material which does not prevent detection of the particles. This can be accomplished, for example, by coating a portion of a sample device with an optically transmissive, solidifying solution.

The present invention presents several advantages over the prior art. These include a reduction of artifacts from particulates and contaminants attached to the surface when the sample with the light scattering particles, protection from physical damage due to handling and exposure to the environment. In addition, using an optically clear coating on a surface that has light scattering debris or surface imperfections such as scratches greatly reduces the background light scattering due to these artifacts. Also, by increasing the index of refraction of the medium surrounding the particle, the scattering efficiency of the particle is increased over air by a factor that corresponds to the fourth power of the refractive index of the medium. For media described in the present invention, this effect approximately doubles the light scattering signal of the particles.

The present invention addresses the needs for labeled sample protection, preservation, and repeat or delayed detection, even after storage for extended periods of time. In addition, when used in conjunction with resonance light scattering particles (RLS particles), the method can also enhance the sensitivity of analyte assays by reducing background scattered light and/or by refractive index enhancement of the scattered light signal. The protection and/or preservation can also be referred to as "archiving"; the medium used for protection and/or preservation can also be referred to as an archiving agent.

As used herein, the term "sample" refers to a material that may comprise an object of interest, e.g., an analyte, as well as one or more labels used in a process of identification or an assay.

As used herein, the term "sample device" refers to a physical item that retains a sample for identification or analysis. Typically, the sample device is configured with surface or surfaces on which sample(s) are retained. Preferably, a plurality of surfaces or zones are available on a sample device for analysis of multiple samples. The term "substrate" is also used to refer to the surface on which the sample and/or label is present. Non-limiting examples of sample devices include slides, chips, plates, microtiter plates, and membranes.

As used herein in connection with sample devices or other items with a solid phase, the term "chip" refers to a substantially planar solid substrate with surface area of 1 in$^2$ or less. Preferably the substrate is optically clear, e.g., glass or plastic although other material supports can be used.

As used in connection with sample devices or other solid phase items, the term "slide" refers to a generally planar solid substrate with a surface area greater than 1 in$^2$ up to 4 in$^2$ inclusive. Preferably the substrate is optically transmissive. Glass microscope slides with dimensions approximately 1 inch by 3 inches are an example. While slides with surfaces that are substantially uniformly planar are preferred, slides may have depressions, ridges, permanently attached or removable well structures, or other surface structures useful or not preventing use of the slide in the intended assay.

Likewise, the term "plate" refers to a solid substrate with a generally planar surface having an area greater than 4 in$^2$. The plate may be substantially uniformly planar, or may have depressions, attached well structures, or other structural features. In some embodiments, the plate has depressions, e.g., wells, for containing liquids, for example, microtiter plates (e.g., 96-well, 192-well, and 384-well plates). In other embodiments, a plate may have either permanently mounted or removable well structures affixed to the surface of the plate.

The term "chamber slide" refers to a slide that has a chambered well or wells on a surface for holding fluid samples during processing, e.g., during incubations. Typically the upper structure defining the well sides is made of polystyrene or the like, and is sealed to the slide surface with an elastomeric gasket, such as a silicon rubber gasket. The gasket and upper structure is generally removable. Thus, individual samples can be applied to different areas of the slide. Typically, but not necessarily, the well structure is removed prior to coating and/or reading the slide.

As used herein, the term "label" refers generally to an entity that is used to identify an object of interest, and in most instances, trace the object through a physical, chemical or biological process. In preferred embodiments, the label is detectable by photons emanating from the label such as resonance light scattering (RLS) particle labels.

As used herein, the terms "scattered light detectable particle", "light scattering particle", and "resonance light scattering particle (RLS)" are used interchangeably to refer to any particle or particle-like substance that is composed of metals, metal compounds, metal oxides, semiconductors, polymers, or a particle that is composed of a mixed composition containing at least 0.1% by weight of metals, metal compounds, metal oxides, semiconductors, or superconductor material. A more detailed description of the particle labels of the invention is provided in Section 5.1.

Various aspects of this embodiment of the invention including the use of membranes are described in details in Sections 5.2, 5.3, 5.4, 5.5, 5.6, 5.7, 5.8 and 5.9 hereinbelow.

## 5.1 <u>RESONANCE LIGHT SCATTERING PARTICLE LABELS</u>

Resonance light scattering (RLS) provide a highly sensitive method for detecting the presence of analytes associated with submicroscopic particles. Preferably the particles are gold and/or silver particles of uniform size, typically in the range of 40-120 nm in diameter, though particles in a greater range can also be used, e.g., 1-500 nm, or 20-200 nm, or 30-300 nm. When illuminated with white or other polychromatic light under appropriate conditions, these particles scatter light of a specific color and intensity, with very high efficiency. The particles can be derivatized with a variety of biomolecules to allow specific particle binding for detection and potentially quantitation of many different target moieties, for example, specific haptens, antigens, proteins, peptides, carbohydrates, lipids, small molecule ligands, nucleic acids, and the like. RLS detection systems also provide excellent spatial resolution for applications requiring precise microscopic localization. Such RLS particles are extremely useful as labels in a variety of analyte assays and are preferred in the methods of the invention.

Thus, in one embodiment, the invention provides a method for preserving a sample comprising light scattering particles or having been contacted with light scattering particles. In certain embodiments, such as negative controls, although the sample has been contacted with light scattering particles, very few or no particles may remain associated with the sample. The preservation of such samples is also encompassed by the present invention. The method of the invention comprises applying a coating composition to at least a portion of the sample to form an optically transmissive coating. The light scattering particles are chosen to be of a size between 1 and 500 nm inclusive, and have properties such that light scattered from one or more of the particles can be detected by a human eye with less than 500 times magnification and without electronic amplification.

Theory of Light Scattering and Selection of Particle Sizes and <u>Compositions That Are Best Suited for Ultra-sensitive Analyte Detection</u>

The optical properties of resonance light scattering (RLS) particles depend on the particle composition, size and shape and refractive index of the bathing medium. The best label compositions and sizes are those which display a strong light scattering band in the visible region of the electromagnetic spectrum (for visual detection applications). The particle compositions and sizes desired for ultra-sensitive detection can be estimated by examination of light scattering theory, especially as expressed by Rayleigh's theory of light scattering. The Rayleigh expression applies to spherical, homogeneous particles that are much smaller than the wavelength of incident light (radius less than about 1/10 of the incident light wavelength). Although some of the particles that are used have diameters that are larger than the Rayleigh size range, the Rayleigh equation nevertheless provides the basic guidance for selection of particles that are best suited for use as ultra-sensitive labels. Before examining the Rayleigh expressions, it is advantageous to understand the mechanism of light scattering which are presented in the following paragraphs.

Mechanism for Light Scattering

When a small particle is illuminated with a beam of monochromatic polarized light (*i.e.*, consisting of electromagnetic waves oscillating in a given direction), an oscillating electric force is exerted on the electrons in the particles. The electrons respond through oscillating in the polarization direction (here taken to be the vertical direction) with the same frequency as the incident light. If the particle is much smaller than the wavelength of the incident light, then all of the electrons in the particle oscillate collectively in phase with the light wave thus producing a large oscillating electric dipole moment. It is known from electrodynamic theory that such an oscillating dipole radiates electromagnetic waves that have the same frequency and wavelength as the driving incident wave. It is this radiation that constitutes the scattered light. It should be stressed that when illuminated with monochromatic light, all particles scatter light at the same wavelength as the incident light, independent of particle size, composition or shape. The light scattering detectable particles can also be configured to display different optical properties, e.g., different colors, under white light illumination as discussed in more detail below.

Theoretical Expressions for Light Scattering and Absorption Spectra and Light Scattering and Absorption Powers of Small Homogeneous Spherical Particles with Different Compositions and Sizes

The Rayleigh equation for small particle scattering can be written as follows for the case where the incident light is polarized along the vertical direction.

$$I_s = \frac{16\pi^4 a^6 n_{med}^4}{r^2 \lambda_0^4} \left| \frac{m^2 - 1}{m^2 + 2} \right| \sin^2 \alpha \quad (1)$$

$I_s$ is scattered light intensity, $a$ is particle radius, $\lambda_0$ is wavelength of incident light as measured in vacuum (the wavelength measured by a spectrophotometer is wavelength in air which, for practical purposes, is the same as wavelength in a vacuum), $n_{med}$ is the refractive index of the medium bathing the particle ($\lambda = \lambda_0/n_{med}$ gives the wavelength of the incident light inside of the bathing medium which is the wavelength sensed by the particle. The $n_{med}$ term adjusts $\lambda_0$ to the wavelength $\lambda$ actually sensed by the particle), $\alpha$ is the angle between the vertical direction of polarization of the incident light and the direction in which the scattering light is detected, r is the distance between the particle and detector, $n_p$ is refractive index of the particle and $m = n_p/n_{med}$ is the relative refractive index of the particle. The refractive index of the particle depends on composition and wavelength and has the same spectrum ($n_p$ vs $\lambda_0$) as the refractive index of the bulk particle material for the particle sizes discussed in this article. The refractive index at different wavelengths for many particle compositions can be found in various handbooks and scientific articles.

The following statements can be made from Rayleigh's equation concerning light scattering properties that are important for the use of RLS particles as ultra-sensitive labels.

1. Scattered light intensity increases very rapidly with increase in particle size. More precisely, it increases with the sixth power of the radius. Thus an 80 nm spherical particle scatters light approximately 64 times more intensely than a 40 nm particle of the same composition.

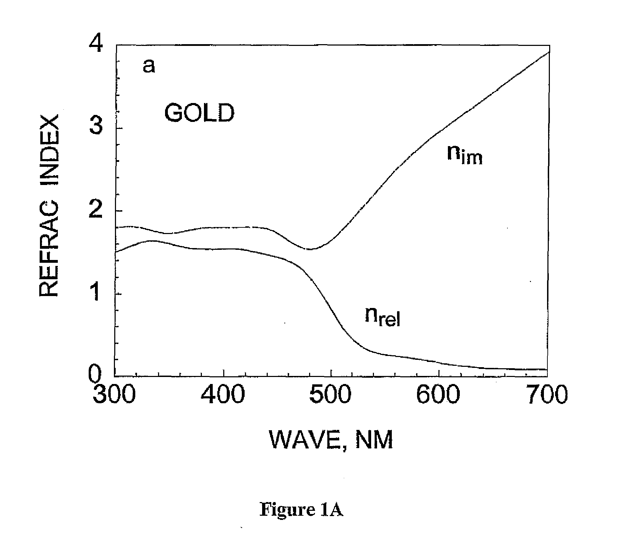

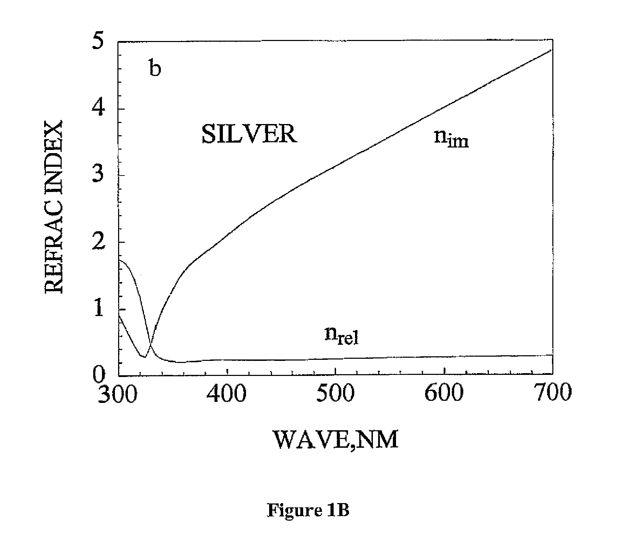

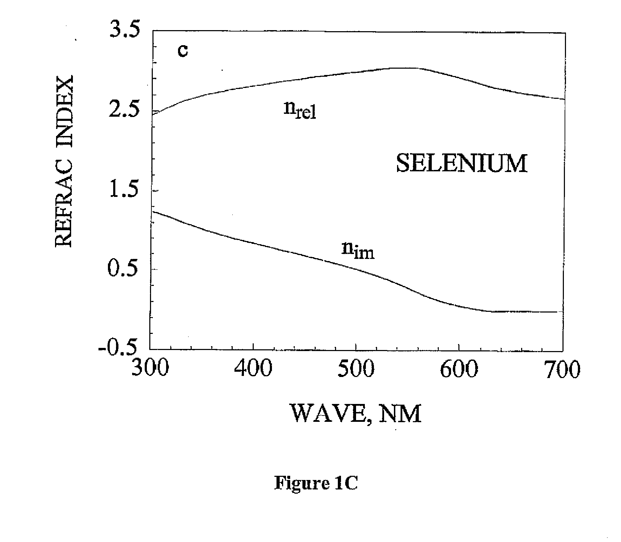

2. The effect of composition on scattered light intensity resides in the term containing the value of m, which is the only parameter in Eq.(1) that depend on composition. To explain how m affects light scattering it is necessary to understand that the Maxwell theory of electrodynamics, on which Rayleigh's equation is based, can account for light absorption only by introducing the concept that refractive index can be a complex number quantity. That is, for materials that absorb light (e.g., display a color in the visible region of the electromagnetic spectrum) the refractive index is a complex number. For materials that are transparent and do not absorb light, the refractive index is a real number. Thus, in general the refractive index $n_p$ of a particle can be expressed as

$$n_p = n_{rel} + i n_{im} \quad (2)$$

where $i = \sqrt{-1}$ and $n_{rel}$ and $n_{im}$ are, respectively, the real and imaginary components of the refractive index. Figures 1A, 1B and 1C shows plots of $n_{rel}$ and $n_{im}$ vs $\lambda_0$ for gold, silver and selenium, respectively. Both $n_{rel}$ and $n_{im}$ depend strongly

on wavelength for these materials. For transparent dielectric materials (visible light) such as glass and polystyrene, $n_{im}$ is zero and usually does not depend strongly on wavelength. For glass $n_{rel}$ = 1.46 (fused quartz) and for polystryrene $n_{rel}$= 1.57-1.60 (depending on the grade). The refractive indices of glass and polystyrene are practically wavelength independent across the visible light wavelengths.

Examination of Rayleigh's equation shows that intense light scattering occurs when the denominator of Eq. (1) is zero, in which case $I_s$ becomes infinitely large. Thus the condition for strong light scattering is that $m^2+2=0$. Solving the latter equation for m gives m=i$\sqrt{2}$ where i=$\sqrt{-1}$. This result indicates that very strong light scattering occurs at any wavelength where the real component of the relative refractive index is zero (i.e., $m_{rel}=n_{rel}/n_{med}=0$) and the imaginary component is equal to $\sqrt{2}$ (i.e., $m_{im}=n_{im}/n_{med}=\sqrt{2}$). For transparent dielectric materials, these conditions cannot be met because for them $n_{im}=0$. Therefore particles composed of, for example, glass and polystyrene are not expected to exhibit strong light scattering signals. However, materials such as metals, metal oxides and semiconductors have complex refractive indices that depend strongly on wavelength and thus have the potential for high light scattering intensity by meeting the strong light scattering condition at some wavelength. These conditions do not have to be met exactly but the closer they are satisfied the stronger is the light scattering band.

Light Scattering Spectra and Light Scattering Powers of Some Particle Compositions

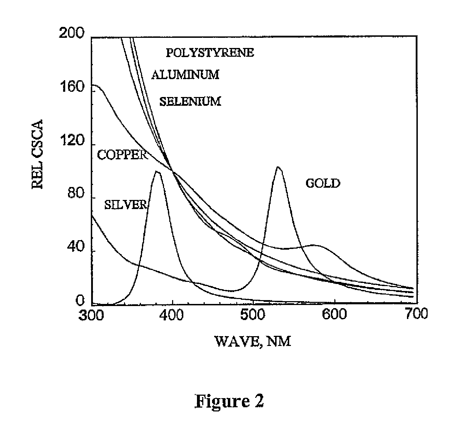

Figure 2 shows the light scattering spectra of 40 nm spherical particles of different compositions, bathed by water, which is calculated using Rayleigh's equation. For glass and polystyrene particles $n_p$ is practically independent of wavelength and $I_s$ vs $\lambda_0$ decreases monotonically with increasing wavelength according to $1/\lambda^4$ as expected from the Rayleigh equation. On the other hand, metal particles can exhibit strong light scattering bands at wavelengths in the visible region due to their complex refractive indices, also known as surface plasmon resonance. Generally, particles that exhibit a strong light scattering band within the wavelengths of 350 - 850 nm, 350 - 450 nm, 400 - 500 nm, 450 - 550 nm, 530 - 640 nm and 600 - 850 nm are preferred. Gold and silver particles can exhibit this surface plasmon resonance in the visible region of the electromagnetic spectrum. These bands are illustrated in the graphs of Figure 2, which show that the conditions for high light scattering are approximately satisfied at 525 nm for the 40 nm gold particles and 380 nm for the 40 nm silver particles. Although selenium has a wavelength dependent complex refractive index, the conditions for strong light scattering are not met at any wavelength in the visible region and 40 nm selenium particles do not display a light scattering band in the visible region.

The amplitudes of the light scattering spectra of Figure 2 have been normalized (so as to present all of the spectra in the same scale) and thus give no information on light scattering power. It is customary to present light scattering power in terms of light scattering cross sections. The light scattering cross section of a particle represents an area around a particle such that any photon of light that impinges in this area is scattered. In the small particle range, the light scattering cross section is given by the expression.

$$Csca = \frac{128\pi^5 a^6 n_{med}^4}{3\lambda_0^4} \left| \frac{m^2-1}{m^2+2} \right|^2 \qquad (3)$$

Values of $C_{sca}$ can range from 0 to values greater than the physical cross sectional area $\pi a^2$ of the particle. The ability of particles to absorb light is also considered. In light scattering theory, absorption refers to a process where a photon of light is removed from the incident light beam and converted into heat. Light scattering and light absorption are independent processes and the sum of the two processes is called extinction. $C_{ext}$ is the quantity measured in an absorption spectrophotometer. More specifically, the amount of light removed from a beam of light as it transverses a light scattering suspension is give by the expression

$$I_t = I_0 e^{-\rho C_{ext} L} \qquad (4)$$

where $I_0$ is incident light intensity, $I_t$ is transmitted light intensity, $\rho$ is particle concentration, $C_{ext}$ is extinction cross section and L is optical path length. The absorption cross section for small spherical particles is given by the expression

$$Cabs = \frac{8\pi^2 a^3 n_{med}^4}{\lambda_0} Im\left(\frac{m^2 - 1}{m^2 + 2}\right) \qquad (5)$$

where Im means the imaginary component of the complex number in parenthesis. The total absorption strength is given by the extinction cross section $C_{ext}$

$$C_{ext} = C_{abs} + C_{sca} \qquad (6)$$

Extinction strength is usually measure in terms of the molar decadic extinction coefficient $\varepsilon$ ($M^{-1}cm$) from measurement of absorbance $A$ using the relation

$$A = Log\left(I_0 / I_t\right) = \varepsilon CL$$ where C is molar concentration and M is moles per liter. $\varepsilon$ and $C_{ext}$ are related by the expression (1)

$$\varepsilon = \frac{N_{av} Cext}{2.303 \times 1000} \qquad (7)$$

$$= 2.63 \times 10^{20} C_{ext} \qquad (8)$$

where $N_{av}$ is the Avogadro's number ($1.602 \times 10^{23}$ $mol^{-1}$). As mentioned, $C_{ext}$ is a measure of the total number of photons removed from a light beam as it passes through a light scattering suspension and photons are removed by both light scattering and absorption. Scattering efficiency can be defined by the expression

$$\varphi_s = \frac{C_{sca}}{C_{ext}} \qquad (9)$$

which gives the fraction of photons removed from a light beam that appear as scattered photons. The scattering strength $C_{sca}$ of a given particle is proportional to the product $\varepsilon \varphi_s$ as shown by the relation

$$C_{sca} = \frac{\varepsilon \varphi_s}{2.63 \times 10^{20}} \qquad (10)$$

The spatial distribution and degree of polarization of scattered light when the illumination is polarized light is also considered. For a small particle suspension that is illuminated with light polarized in the vertical direction, the intensity is highest in any direction perpendicular to the direction of polarization ($\alpha$=90° in Eq. 1) and independent of azimuth angle (independent of rotation about the vertical direction. Furthermore, the scattered light is completely polarized in the vertical direction. That is, if the scattered light is viewed through a polarizer, the intensity is high when the polarizer is oriented in the vertical direction and zero when the orientation is changed to the horizontal direction. If the orientation of the detector is rotated from $\alpha$=90° to $\alpha$=0°, the scattered light intensity decrease as $\sin^2(\alpha)$ as $\alpha$ is decreased and becomes zero at 0°. These effects can be easily seen experimentally. If the particle is illuminated with unpolarized light, then polarization effects are diminished.

Particles Larger than Wavelength of Incident Light

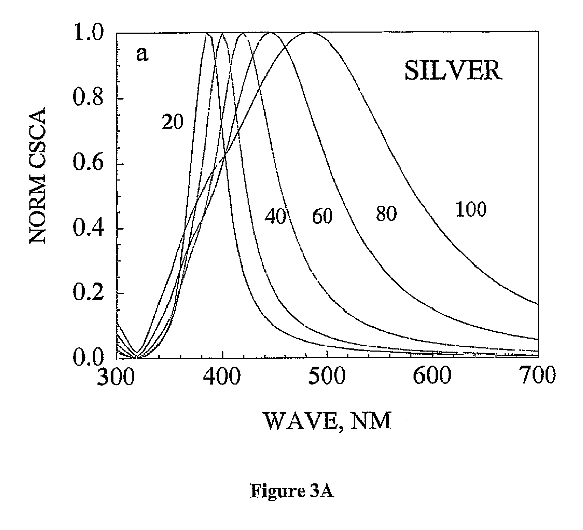

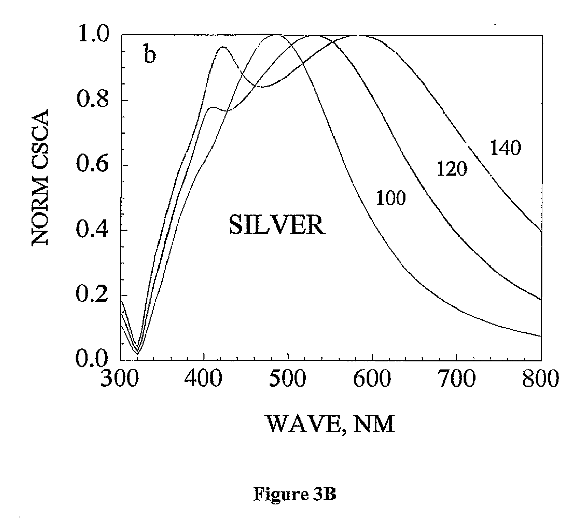

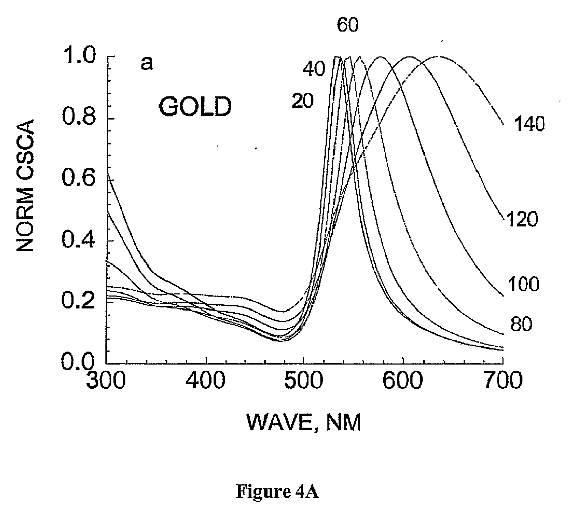

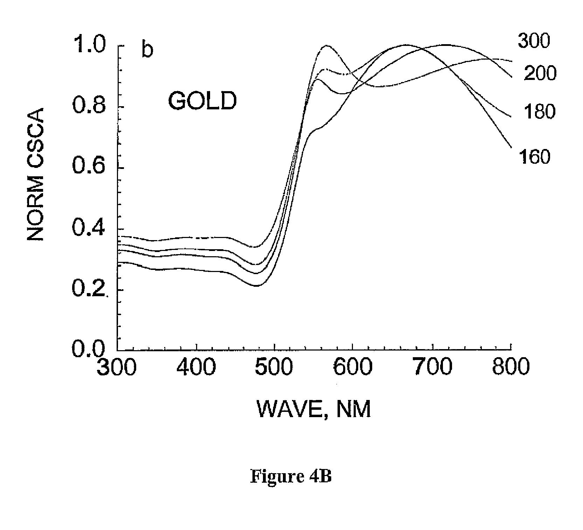

The light scattering and absorption properties of particles with diameters that are comparable or larger than the wavelength of incident light cannot be understood in terms of Rayleigh theory but can be predicted by Mie theory, which is more complex than Rayleigh theory and thus will not be presented here. One of the main changes in light scattering properties that occur in the large particle range is that the color of the scattered light changes with increasing particle size or, more fundamentally, the light scattering band shifts towards higher wavelengths with increase in particle size. In the small particle range, scattered light intensity increases with particle radius but the shape and wavelength maximum of the light scattering and absorption bands do not change with change in particle size. Figures 3 and 4 and show light scattering spectra of different size silver and gold particles calculated with Mie theory. It has been found that predictions of Mie theory have been found to agree very well with experimental results. For ultra-sensitive detection, light scattering cross sections around $10^{-10}$ cm$^2$, or values of $\varepsilon\varphi_s$ around $2.5\times10^{10}$ M$^{-1}$ cm$^{-1}$, are preferred.

The spectral changes which occur with increase in particle size in the large particle range can be explained qualitatively as follows. In the small particle range, all of the electrons in a particle oscillate with the same phase and give rise to a large oscillating dipole moment. For large particles (diameter greater than about 40 nm), the electrons in different parts of the particle oscillate with different phases since they sense different phases of the incident light wave. Light waves scattered from different regions of the particle have different phases and thus interfere at the surface of the particle. It is this interference that results in changes in scattered light spectrum as particle size is increased.

Formulae Developed for Studying Particle Light Scattering Parameters

One skilled in the art can use the theoretical methods of the present invention to evaluate, modify, and adjust specific particle parameters of composition, size, shape, and homogeneity to derive (*i.e.*, configure) one or more desirable light scattering properties that are easily detected and measured. Considerations need to be made with regard to sample types, diagnostic formats, and limitations of apparatus illumination and detection means in the choice of particles. For example, in one application, multi-analyte detection may be performer on a solid-phase sample that contains a high non-specific light background on a high throughput testing apparatus, while in another application, single analyte detection in solution is performed in a point of care assay in a doctor's office.

The main objective is to optimize particle types for use in analytical and diagnostic assays. In many of the applications, the particles is coated with a macromolecular substance such as polymer, protein, or the like to confer suitable chemical stability in various mediums, as is known in the art. Binding agents such as antibodies, receptors, peptides, proteins, nucleic acids, and the like can also be placed on the surface of the particle so that the coated particle can be used in an analytic or diagnostic format. In some applications, the binding agent serves a dual function in that it stabilizes the particle in solution and provides the specific recognition binding component to bind the analyte. The coating of particles with proteins such as antibodies is known in the art. However, the main interest is in measuring one or more specific parameters of the light scattering signals of different types of particles which in some cases are of similar size and/or shape and/or composition and it is desired to determine the optical resolvability of one or more of the specific light scattering properties of coated particles.

It has been determined by physical experimentation and theoretical modeling that the presence of thin coats of binding agents, non-optically absorbing polymers (in the visible region of the spectrum), or other materials on the particle surface does not noticeably alter the light scattering properties specific for that type of particle which is not coated with these types of materials. By "thin coat" is meant monolayer(s) of different amounts and compositions of the above materials coated on the surface of the particle.

Specific Light Scattering Properties of Particles

A brief summary of some of the most important light scattering properties that can be used to detect analytes in various sample types using a variety of different assay formats is presented. The measured light scattering properties that are detected are one or more of the following: the intensity, the wavelength, the color, the polarization, the angular dependence, and the RIPSLIW (rotational individual fluctuations in the scattered light intensity and/or wavelengths) of the scattered light of the scattered light.

Coated and uncoated metal-like particles have similar light scattering properties and both have superior light scattering properties as compared to non-metal-like particles. In addition, it has been determined that it is relatively easy to adjust the types of light scattering properties in metal-like particles by varying in one form or another, the size, shape, composition, and homogeneity such that the specific light scattering attributes can be measured from the metal-like particle in various sample types.

Metal-like particles can be detected to extreme sensitivity. The individual particles can be easily detected to the single particle limit with inexpensive and easy to use apparatus, such as, through a method of illumination and detection termed DLASLPD (direct light angled for scattered light only from particle detected), which is disclosed in United States Patent No. 6,214,560.

One or more types of metal-like particles are detected in a sample by measuring their color under white light or similar broad band illumination with DLASLPD type illumination and detection methods. For example, roughly spherical particles of gold (for example, coated with binding agent, bound to analyte, released into solution or bound to a solid-phase) of 40, 60, and 80 nm diameters and a particle of silver of about 30nm diameter can easily be detected and quantitated in a sample by identifying each particle type by their respective unique scattered light color and/or measuring the intensity. This can be done on a solid phase such as a microtiter well or microarray chip, or in solution. The measurement in solution is more involved, because the particles are not spatially resolved as in the solid-phase format. For example, one can detect the different types of particles in solution by flowing the solution past a series of detectors each set to measure a different wavelength or color region of the spectrum and the intensity at these different wavelengths is measured. Alternatively, a series of different wavelengths of illumination and/or detection can be used with or without the flow system to detect the different particle types.

For solid-phase analytical applications, a very wide range of concentrations of metal-like particles is detectable by switching from particle counting to integrated light intensity measurements depending on the concentration of particles. The particles can be detected from very low to very high particle densities per unit area.

In other assay applications, the particles which are bound to a solid substrate such as a bead, surface such as the bottom of a well, or the like can be released into solution by adjusting the pH, ionic strength, or other liquid property. Higher refractive index liquids can be added, and the particle light scattering properties are measured in solution. Similarly, particles in solution can be concentrated by various means into a small volume or area prior to measuring the light scattering properties. Again, higher refractive index liquids can be added prior to the measurement.

Small nonspherical particles behave somewhat as linear dipole scatters with the absorption and emission moments along the long axis of the particle. The following observations have been made under DLASLPD illumination and detection conditions in an ordinary light microscope. When the illuminating light is linearly polarized, the non-spherical particles flicker as they rotate. The particles are most intense when their orientation is such that their long axis is oriented in the direction of polarization and is at a minimum when the moment is perpendicular to this direction. In contrast, small spherical particles do not flicker when illuminated by polarized light. For nonspherical particles of certain compositions, the color of the scattered light (with white light illumination) changes with the degree of asymmetry. As the asymmetry is increased, the color shifts towards longer wavelengths. For example, asymmetric particles of silver have been observed to change colors as the particles were rotating in solution when viewed with an ordinary light microscope under DLASLPD like conditions (RIFSLIW). This rotational property is used in many different aspects of the current invention to more specifically and more sensitively detect and or measure one or more analytes or particles in a sample.

It has also determined that certain mixed compositions of particles made from metal-like materials, and non-metal-like and metal-like materials provides for additional light scattering properties and/or additional physical properties. These properties include the ability to manipulate the particles by applying an electromagnetic field (EMF). This property of the particles can be used in many different ways with the practice of one or more aspects of this invention. Illustrative discussions of particle-dependent light scattering properties and the use of these properties to detect one or more analytes in a sample is now provided.

It will be useful to first describe the present invention in terms of the light scattering properties of homogeneous, spherical particles of different sizes and compositions. However, the basic aspects of the invention apply as well to non-spherical particles as one in the art can determine. In addition, it will be useful to describe the present invention in terms of the incident light wavelengths in the range 300nm to 700nm. However, the basic aspects of the invention apply as well to electromagnetic radiation of essentially all wavelengths. By "light" is meant ultraviolet, visible, near infrared, infrared, and microwave frequencies of electromagnetic radiation. It will be further useful for the description of the present invention to use polystyrene particles to represent non-metal-like particles of various types. Other non-metal-like particle types include those composed of glass and many other polymeric compounds. Such particles have roughly similar light scattering characteristics as compared to polystyrene particles.

The relative intensities of scattered light obtained from different particles irradiated with the same intensity and wavelengths of incident light can be directly compared by comparing their $C_{sca}$'s. The higher the $C_{sca}$, the greater the scattering power (light scattering intensity) of the particle. In the following sections the words "scattering power" are used to mean $C_{sca}$ or scattered light intensity.

We have calculated the light scattering powers, in water, of small spherical particles identical in size, and different in composition, for incident wavelengths over the wavelength ranges of 300 to 700 nanometers (nm). The values of refractive index vs. wavelength (in vacuum) for the different bulk materials used in these calculations can be found in standard handbooks.

For some particle compositions, the light scattering power decreases continuously from 300 to 700 nm while for other compositions the scattering power vs. wavelength profile shows peaks or bands. When these peaks or bands are in the visible region of the spectrum the light scattered by the particles is colored when the incident light is white.

Figure 4A shows the calculated scattered light intensity versus incident light wavelength spectra profiles for spherical gold particles of varying diameter. The scattered light intensity peak wavelengths shift to longer wavelengths as the size of the gold particles is increased. These light scattering properties for coated or uncoated gold particles of 40, 60, 80, 100 nm diameters are similar and they appear as green, yellow-green, orange, and orange-red particles when illuminated with a white light source. Small spherical silver particles appear blue (Figure 3A). Thus, metal-like particles coated with various types of binding agents can be used in numerous ways in analytic type assays. The configurable properties of scattered light detectable particles, e.g., the color of different types of metal-like particles, allows for multi-analyte detection. Different populations of light scattering particles can be used in the detection of different types of analytes in a multi-analyte assay (*i.e.*, multiplex assay), where each population of particles used for the detection of a particular type of analyte is configured to emit scattered light that is distinguishable from that of any other populations of particles. For example, spherical gold particles of 40, 60, 80, and 100 nm diameter and 20 nm diameter silver particles, each coated with a different type of binding agent, can be used in the same sample to detect five different analytes in the sample. In one format, five different types of cell surface receptors, or other surface constituents present on the cell surface can be detected and visualized. Detection of the scattered light color of the differently coated particles that are bound to the surface of the cell under DLASLPD conditions with a light microscope with white light illumination makes this possible. The number and types of analytes are identified by the number of green, yellow, orange, red, and blue particles detected. Similarly, chromosome and genetic analysis such as in situ hybridization and the like can also be done using the method as described above where the different types of metal-like particles are used as "chromosome paints" to identify different types of nucleic acid sequences, nucleic acid binding proteins, and other similar analytes in the sample by the color of the scattered light of the different types of metal-like particles. These examples are provided as illustrative examples, and one skilled in the art will recognize that the color of the scattered light of different types of metal-like particles can be used in many different assay formats for single or multi-analyte detection.

Thus, adjusting the size of certain types of spherical metal-like particles is a useful method to increase their detectability in various samples by using the color and/or other properties of their scattered light. By using a white light source, two or more different types of particles are easily detectable to very low concentrations.

Mixed Composition Particles

Spherical particles of mixed compositions were evaluated by theoretical and physical experimentation to assess their possible utility in various diagnostic and analytic applications. For theoretical evaluations, a gold "core" particle coated with different thickness of silver and a silver core particle coated with different thickness of either gold or polystyrene were studied. By "core" is meant a spherical particle upon which an additional layer or thickness of different light scattering material is placed, resulting in a mixed composition of certain proportions. Direct physical experimentation was done for particles composed of a mixed composition where an additional thickness of silver was added to a core gold particle of 16nm diameter. In these illustrative examples, gold and silver are representative of metal-like materials and polystyrene is representative of non-metal-like materials. These examples are only a few of a larger number of different possible combinations which involve particles composed of mixtures of one or more different metal-like and/or non-metal-like materials.

For particles composed of certain mixed compositions of metal-like materials, as for example, mixed compositions of gold and silver, new light scattering properties appear which are useful in many different sample types and specific diagnostic and analytic applications. Particles with two or more optically distinct and resolvable wavelengths of high scattering intensities can be made by varying the composition of the metal-like-materials.

In contrast, particles composed of mixed compositions of non-metal-like and metal-like materials generally exhibit light scattering properties similar to the metal-like materials at equal proportions or less of non-metal-like materials to metal-like materials. Only at very high proportions of non-metal-like to metal-like materials do the light scattering properties of the mixed composition particle resemble that of the non-metal-like material as the results in section B of Table 1 indicate.

Both the mixed silver-gold compositions and the silver-polystyrene compositions exhibit the high light scattering power and visible wavelength scattering bands which are characteristic of particles composed of pure metal-like materials. Particles of certain mixed compositions are detectable by specifically detecting the scattered light from one or both of the scattering intensity peaks and or by the color or colors of these mixed composition type particles. Such mixed composition type particles enhances the capability for detecting lesser amounts of particles and more specifically, detecting lesser and greater amounts of particles than was previously possible.

**Table 1**

| | | PARTICLE DIAMETER | SILVER CORE DIAMETER | COAT COMPOSITION | COAT THICKNESS | <u>VOL SILVER</u> TOTAL VOL | $C_{sca}$ AT WAVELENGTH MAXIMA ($cm^2$) | INCIDENT WAVELENGTH SCATTERING MAXIMA |
|---|---|---|---|---|---|---|---|---|
| | | **CALCULATED SCATTERING PROPERTIES OF SPHERICAL <u>MIXED COMPOSITION PARTICLES - SILVER CORE PARTICLE</u> COATED WTTH GOLD OR POLYSTYRENE (PST)** | | | | | | |
| <u>A</u> | | 10 nm | 10nm | - | 0 | - | $1.1 \times 10^{-14}$ | -384nm |
| | | 14 nm | 10nm | Gold | 2nm | 0.36 | $4.3 \times 10^{-15}$ | ~372nm |
| | | 20 nm | 10nm | Gold | 5nm | 0.125 | $6.5 \times 10^{-15}$ | ~525m |
| <u>B</u> | | 10nm | 10nnm | - | 0 | 1 | $1.1 \times 10^{-14}$ | ~384nm |
| | | 10nm | 9nm | Gold | 0.5nm | 0.73 | $1.9 \times 10{-15}$ | ~384nm |
| | | 10nm | 7nm | Gold | 1.5nm | 0.34 | $5.6 \times 10^{-16}$ | ~300nm |
| | | | | | | | $6.8 \times 10^{-16}$ | ~520nm |
| <u>CI</u> | | (a)10nmPST | 0 | - | 0 | - | $1.3 \times 10^{-"}$ | ~300nm |
| | | 10nm | 10nm | - | 0 | 0 | $1.1 \times 10^{-14}$ | ~384nm |
| | | (a)20nmPST | 0 | - | 0 | - | $8.3 \times 10^{-16}$ | ~300nm |
| | | 20nm | 20nm | - | 0 | 1 | $7 \times 10^{-13}$ | ~382nm |
| | | 40nm | 20nm | PST | 10nm | 0.125 | $9.3 \times 10^{-13}$ | ~412nm |
| | | 60nm | 20nm | PST | 20nm | 0.037 | $1.25 \times 10^{-12}$ | ~418nm |
| | | 20nm | 12nm | PST | 4nm | 0.216 | $4 \times 10^{-14}$ | ~408nm |
| | | 20nm | 10nm | PST | 5nm | 0.125 | $1.4 \times 10^{-14}$ | ~410nm |
| | | 20nm | 8nm | PST | 6nm | 0.064 | $4.3 \times 10^{-13}$ | ~414nm |
| (a) Particle composed of polystyrene only | | | | | | | | |

Asymmetric Particles or Non-spherical Symmetric Structures

The physical orientation of non-spherical particles, such as asymmetric or symmetric non-spherical particles with regard to an incident light beam allows for additional scattered light properties to be used in the detection of these particles. Non-spherical symmetric structures include oblate spheroids, triangular, or hexagonal particles, rods, or other polygonal particle structures, and cylindrical structures including rods, cylinders, cones etc. The characteristics of the light (such as color, wavelength, polarization, etc.) scattered by a non-spherical structure is highly dependent on its geometry and its orientation relative to the polarization of the illuminating light beam. This unique property is responsible for the observation of rotational individual fluctuations in the scattered light intensity and or wavelengths (RIFSLIW).

Small non-spherical particles (whether symmetric or asymmetric) behave somewhat as linear dipole scatterers with different absorption and emission moments along the long or major axis of the particle as compared to the minor axis. The following observations have been made under DLASLPD illumination and detection conditions in an ordinary light microscope. When the illuminating light is linearly polarized, unbound or weakly bound non-spherical particles flicker as they move (*e.g.*, by rotation). The particles are most intense their major axis is oriented in the direction of polarization of the light and is at a minimum when the moment is perpendicular to this direction. In contrast, small spherical particles do not flicker when illuminated by polarized light. For non-spherical particles of certain compositions, the color of the scattered light (*e.g.*, under white light illumination) changes with the degree of asymmetry. As the asymmetry is increased, the color shifts towards longer wavelengths. For example, asymmetric particles of silver were observed to change colors as the particles were rotating in solution when viewed with an ordinary light microscope under DLASLPD like conditions. RIFSLIW is used in many different aspects of the current invention to more specifically and more sensitively detect and or measure one or more analytes or particles in a sample.

Applicant has also determined that certain mixed compositions of particles made from metal-like materials, and non-metal-like and metal-like materials provides for additional light scattering properties and/or additional physical properties. These properties include the ability to manipulate the particles by applying an EMF field. This property of the particles can be used in many different ways with the practice of one or more aspects of this invention. Further illustrative discussions of particle-dependent light scattering properties and the use of these properties to detect one or more analytes in a sample are now provided.

The property of RIFSLIW can be used in many different aspects of the current invention to more specifically and more sensitively detect and or measure one or more analytes or particles in a sample. For example, the flickering of the scattered light intensity and/or change in color provides additional detection means to determine which particles are bound to a surface and which particles are not. This allows for non-separation type of assays (homogeneous) to be developed. All that is required is to detect by particle counting, intensity measurements or the like the particles that do not flicker and/or change color. Unbound particles in solution will flicker and/or change color while those bound to the surface will not. Additional image processing means such as video recorders and the like allow for additional methods of detection to be used with both asymmetric and spherical (symmetric particles). For example, in either a separation or non-separation format, the bound particles are detected by focusing the collecting lens at the surface and only recording those scattered light signals per unit area which are constant over some period of time. Particles free in solution undergoing Brownian motion or other types of motion results in variable scattered light intensity per unit area per unit time for these particles. Bound light scattering particles are fixed in space and are not moving. By using image-processing methods to separate the "moving" light-scattering particles from the "bound" light scattering particles, the amount of bound particles is determined and correlated to the amount of analyte in the sample. One of skill in the art will recognize there are many other image processing methods that can be used to discriminate between bound particles to a surface and unbound spherical or asymmetric particles in solution.

Addition of Other Materials to the Surface or Core of the Particle to Provide Additional Physical Attributes not Related to the Light Scattering Properties

In certain applications and with the use of certain types of compositions, it may be useful to "coat" the surface of a particle to further chemically stabilize the particle, or to add additional surface binding attributes which can be very important in specific applications to analytical diagnostic assays. For example, it is well known that silver rapidly oxidizes. For use of silver particles or particles of mixed composition which contain silver, one can chemically stabilize the silver-containing particle by applying a thin coat of gold or other substance on the surface such that the silver is no longer susceptible to environmental effects on it's chemical stability.

In another example, one may want to coat the surface with another material such as a polymer containing specifically bound binding agents, or other materials useful for attaching binding agents, or the binding agents themselves to the particles. In each of these examples, these "thin" coats do not significantly alter the light scattering properties of the core material as the light scattering concentration of these materials is negligible relative to scattering from the gold/ silver particles. By "thin" coats is meant a monolayer or similar type of coating on the surface of the particle.

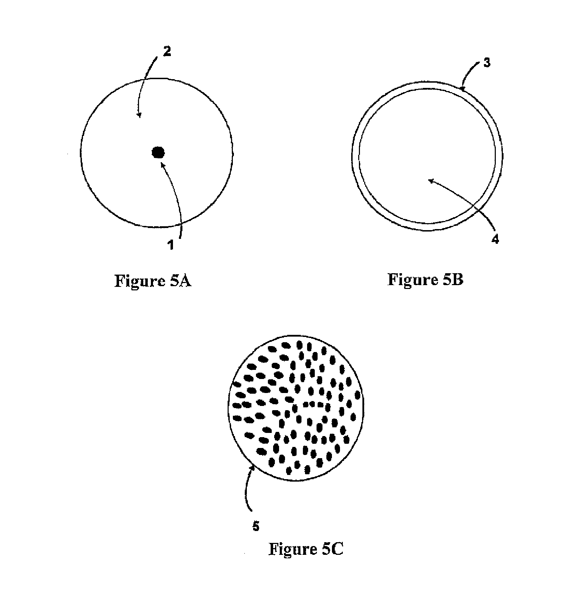

Manipulatable Light Scattering Particles (MLSF's) are particles which in addition to having one or more desirable light scattering properties, these particles can also be manipulated in one-, two- or three-dimensional space by application of an EMF. A MLSP particle can be made in many different ways. For example, a MLSP particle is made by coating a small diameter "core" ferro electric, magnetic or similar material with a much greater proportion of a material that has the desirable light scattering properties, for example a 10nm diameter core of magnetic or ferro electric material is coated with enough gold to make a 50, 70, or 100 nm diameter particle. This is shown in Figure 5A.

Another method of making such a particle is to coat the material that has the desirable light scattering properties with a thin coat of the magnetic or ferro electric material. For example, a gold or silver particle of about 50 nm is coated with a 1-2 nm thick coat of the magnetic or ferro electric material. This is shown in Figure 5B.

Alternatively, the MLSP particles are made by mixing in the appropriate proportions the light scattering desirable materials and the ferro electric or magnetic materials such that as the particle is formed, the appropriate proportions of light scattering desirable material to magnetic or ferro electric material per particle ratio is attained. This is shown in Figure 5C.

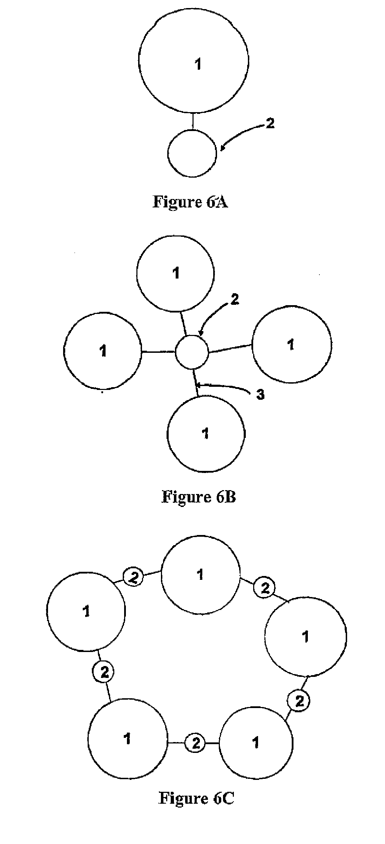

An alternative to the above MLSP particles is to link or assemble one or more types of particles with desirable light scattering properties to one or more particles that can be moved by a EMF. Such multi-particle structures can then have similar properties to the MLSP's. For example, small particles of magnetic or ferro electric material are linked to one or more particles who's light scattering properties are detected. The linking is by ionic, chemical or any other means that results in a stable multi-particle structure. For example, the different particles are coated with appropriate polymers so that when mixed in the proper portion, a defined distribution of discreet multi-particle structures are achieved by crosslinking the different types of individual particles together. There many different ways to link the particles together to achieve the desired multi-particle structure(s). For illustrative purposes, a few of the possible multi-particle structures are shown in Figures 6A, B, and C, which show dimer, tetramer, and higher order particle constructs, respectively, for orientable MLSP particles. It is also envisioned that the multi-particle structure can be formed from a linear arrangement of two or more particles. One skilled in the art will recognize that these are just a few of the many different types of multi-particle structures possible and there are numerous methods to make such structures.

These examples of particles composed of mixtures of one or more material are but a few of a very large number of different compositions of different materials which are possible, and which would be apparent to one of skill in the art.

Particle Size and Shape Homogeneity

Depending on how the light scattering properties of particles are detected, the approximate size and distribution of particle sizes in the particle population can be extremely important. As an example, many of the commercially available gold particle preparations quote the particle size distributions any where from about <10 to about <20 percent coefficient of variation. Percent coefficient of variation is defined as the standard deviation of the particle size distribution divided by the mean of the particle preparation. Thus, for a 60nm particle preparation with a coefficient of variation of 20%, one standard deviation unit is about +12nm. This means that about 10% of the particles are smaller than 48nm or greater than 72nm. Such variation in size has significant effects on the intensity of scattered light and the color of scattered light depending on the approximate "mean" size of the particles in the preparation.

Populations of particles that display a narrow size distributions are preferred. A preferred procedure for making such particles involves first making a preparation of "seed" gold particles which is then followed by taking the "seed" particle preparation and "growing" different size gold or silver particles by chemical methods. For example, 16nm diameter gold particles are used as the "seed" particle and larger diameter gold particles are made by adding the appropriate reagents. This method is also very useful for making mixed composition particles. For examples of these particle preparation methods, *see* U. S. Patent No. 6,214,560.

Particle Homogeneity - Detection of Analytes by Scattered Light Color of Individual Particles

In certain applications, the color of the individual particles are used to identify and quantitate specific types of analytes. For example, in image cytometry applications, it may be of interest to identify and count different types of cell surface antigens or the like by detecting the number and color of different types of particles attached to the surface. For this or any other related type of multi-analyte detection, the size distributions of the different particles need to be kept as tight as possible. The average particle diameter of the particle preparation should be chosen to provide the desired color of scattered light under white light illumination, using an average or "mean" particle size that is as close to the size midpoint between the mean particle sizes of smaller and larger particles which will be used in the same application to produce different colors of scattered light. In this fashion, the resolvability of the different types of particles by their color of scattered light is maximized.

Particle Homogeneity-Integrated Light Intensity Measurement

The intensity of scattered light can vary greatly as particle size is increased or decreased. This variation in the intensity must be taken into consideration especially when integrated light intensity measurements are being performed. Using the 60nm particle preparation described above with a 20% coefficient of variation, this means that 10% of the particles have intensities about 3 times greater or less than a 60 nm particle. In addition, the particles within the remaining 90% of the population have quite varying intensities. In applications where there are many particles being measured, the "average" integrated light intensity should approximate a 60nm particle. However, at lower concentrations of particles, the statistics of such a variation may affect the accuracy of the reading from sample to sample, and correction algorithms may be needed. By using the narrowest distribution of particles possible, the accuracy and ease of measurement is enhanced.

Useful Metal-like Particles for Detection of Analytes by their Light Absorption Color

For some types of analyte assays, analytes are at concentrations where detection of the analytes by the light absorption properties can be accomplished. For example, a current problem in the art of immuno-chromatographic assays and the like is that the use of gold particles of the sizes typically used (4 to 50 nm diameter) only provides for particles that can not be optically resolved by their light absorption color. These particles have a pink to red color when observed on filter paper or similar diagnostic assay solid-phase media. By varying the size and/or shape of silver particles and other metal-like particles many different colors of light absorption can be achieved. These different colors of the particles by light absorption can be used to detect different analytes by the light absorption color of a particle. These colors which can be detected by the eye are very useful in many types of solid-phase assays such as immuno-chromatographic flow assays, panel type, and microarray or larger solid-phase single or multi-analyte assays. Spherical and asymmetrical particles of silver and certain mixed compositions of other metal-like particles allow for a wide range of colors by light absorption.

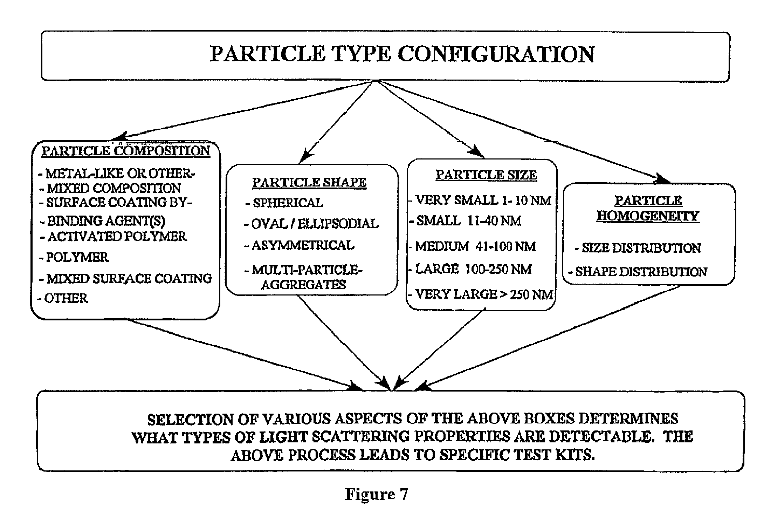

One skilled in the art can practice many different aspects of this invention by using various particle types, with many different particle type configurations, in order to achieve a desired diagnostic or analytic detection capability. Figure 7 shows how one skilled in the art would choose the appropriate particle composition, shape, size and homogeneity to suit a specific diagnostic analytic testing need with detection of the desired light scattering properties of the particles.

## 5.2 SAMPLE PRESERVATION

The invention is described herein with emphasis on uses with respect to resonance light scattering (RLS) particle labels, however, the invention is not so limited as it can also be used with other types of labels that emanate visible light or other types of EM radiation. Examples of other types of labels that can take advantage of this aspect of the invention include fluorescent labels, luminescent labels, chromogenic labels, and radioactive labels, among others. Analyte assays and sample devices that employ such labels are well-know to those of ordinary skill in the art. Analyte assays that use RLS particle labels in combination with these types of labels in the same sample are also contemplated. In certain embodiments of the invention, depending on the configuration of the analyte assay, the labels are attached to one or more analytes of interest. Different types of labels can be used to trace or identify different analytes or analytes from different sources.

In one embodiment, the present invention provides a method for preserving a sample that comprises light scattering particles, or that has been contacted with a composition comprising light scattering particles. The method comprises coating or covering at least a portion of the sample with at least one optically transmissive coating that allows detection of light scattered by the light scattering particles present on the sample. In various embodiments, the invention provides a sample device that facilitates detecting the presence or amount or both of analytes on a sample having light scattering particle labels bound with analytes attached thereto. The sample on or in the sample device is illuminated and in the presence of a coating, light scattered from the labels are detected which serves as an indication of the presence and/or amount of analytes that are present on the sample. In certain embodiments, the covering or coating is reversible, i.e., the removal of the covering or coating does not physically and/or chemically alter the sample or label and/or its position relative to other samples, labels in the sample or sample device. In others, it is irreversible. Typically, a sample comprising labels is present on a solid phase and a single optically transmissive coating material is layered on top of the sample and the solid phase, such that a barrier is formed between the sample and the atmosphere above it. The invention also provides a preserved sample device, which includes a solid phase with light scattering particle labels attached, and an optically clear solid coating covering the light scattering particle labels. In most cases, the light scattering particles labels are associated, directly or indirectly, with analytes or samples on the solid phase.

Examples of coating materials that may be used in the present invention include without limitation, terpenes, polyurethanes, polyesters, acrylics, lacquers, epoxide polymers, polyvinylalcohol, carbohydrate or other biopolymers, organic polymers, aqueous polymers, monomer chemical units that can be solidified via polymerization or cross-linking, as well as chemically and optically compatible combinations and copolymers thereof. Use of the present invention confers a number of advantages including but not limited to one or more of the following: reduction of background signal, enhancement of light scattering efficiency, sample immobilization and protection, label immobilization and protection, convenience in handling and storage, and performance characteristics that allow repeated or postponed analysis with consistent results.

However, the sample device can also be preserved using other techniques, for example, by covering at least a portion of the device with a coating composition that is itself covered with a small optically clear plate, e.g., a plastic, glass, or quartz crystal coverslip or the like. The small plate can be held in place with by surface tension of the solution and/or viscosity of the solution (the solution can act effectively as a glue). The composition may have high viscosity both before and after application (though still sufficiently fluid to cover the sample device without void, or may become more viscous following application on the sample device. Likewise, at least a portion of the sample device can be covered with a solution that sets up to form a network or gel, for example, polyacrylamide and agarose gels. The network or gel can be covered by a small plate as described above. The plate can be held in place via surface tension and/or by some degree of bonding between the plate and the network or gel.

For embodiments in which a non-solidifying composition is used, the preservation may be shorter term than for embodiments in which a solidifying composition is used, due to drying (especially around the edges of a covering plate). However, in such cases, the preservation can be extended by sealing the non-solidifying solution, thereby significantly slowing the evaporation rate (i.e., reducing the evaporation rate by at least 50%, 70%, 80%, 90%, 95%, or more as compared to the non-sealed case) or effectively stopping evaporation (e.g., slowing the evaporation rate to less than 5%, 3%, 2%, 1%, 0.5% or even less as compared to the non-sealed case). Such sealing can involve covering the non-solidifying compsoition (and the covering plate if present) with a layer of an additional optically clear material with low permeability to the solvent or solvents that would otherwise evaporate from the solution to produce the reduced evaporation rate. Alternatively, in cases where the non-solidifying solution is covered with a small plate, the seal maybe only around the edges of the plate. In this case, the sealing material maybe, but need not be, optically clear, i.e., enclosing the non-solidifying solution in an optically clear enclosure.

One practical problem encountered in applying RLS technology on solid surfaces or membranes is that dust, particulate contaminants, surface irregularities or optical properties of the underlying substrate that scatter light will contribute to a non-specific background signal. That background signal may obscure the primary scattering signal from the label particles. Thus, in one embodiment, the invention provides a method of preserving a sample comprising covering or coating the sample with a material that prevents particulate matters from co-mingling with labels, such as light scattering particles, in the sample.

As it is observed that the scattering efficiency of scattered light detectable particles depends on the refractive index of the material surrounding them, it is preferable to use a material with a refractive index that both enhances RLS particle scattering and suppresses non-specific background scattering. With a higher refractive index material such as water, as compared to air, a stronger signal is produced. Liquids that have these properties have been described (e.g., Yguerabide & Yguerabide, 1998, *supra*.) However, liquids tend to be messy to use and to be susceptible to evaporation and contamination. As a result, a liquid that can be solidified or hardened to form an impermeable surface and that can transmit light is preferred in many applications. Accordingly, in another embodiment, the invention provides a method of preserving a sample comprising covering or coating the sample with a material that can be changed from a liquid phase to a solid phase, and preferably the solid phase has a higher refractive index relative to air and/or water. Therefore, the solid coating provides reduction of background and/or specific light scattering enhancement from the particles. In preferred embodiments, the optically transmissive coating is an optically transmissive solution that solidifies on the sample or sample device. In certain embodiments, multiple coatings of the same or different optically transmissive materials can be applied.

In addition to background reduction and/or specific light scattering enhancement, a solid coating can provide physical and/or chemical protection for labeled samples. In this respect, the ability to achieve precise spatial localization with RLS is particularly useful for cell biology, molecular biology, and analytical chemistry applications in which the analyte/target to be detected is immobilized on a solid surface. For example, the RLS labels can be present on a sample such as tissues, histological sections, whole cells, sub-cellular components, chromosome preparations or microarrays with a plurality of spatially-specific features. In preferred embodiments, the sample device includes a solid phase array. Likewise, in preferred embodiments, the sample device includes a slide, a chamber slide, a microtiter plate, an array chip, a membrane, or the like. If the binding of the particles to their analytes/targets or to the surface is accomplished via a chemical reaction or surface adherence, it is susceptible to reversal. Accordingly, the invention provides a method for preserving a sample comprising covering or coating the sample with a solid phase that is impermeable to damaging liquids or gases and/or that is resilient against physical forces that might damage the sample and/or labels or dislodge the labels from their original location on the sample or sample device. For example, the compositions and methods can be used to fix the locations and/or spatial orientation of RLS labels relative to the sample on a solid phase in substantially irreversible manners.

Furthermore, samples that are analyzed by RLS detection can potentially be re-analyzed repeatedly for many times (potentially effectively an infinite number of times), providing essentially the same quantitative output of scattered light each time (with the same illumination conditions). This is because the RLS signal does not quench, fade, decay, or bleach, as does fluorescence, chemiluminescence, radioisotopes and many chromogenic detection systems.

While the methods of the invention can be used for repeated and/or postponed analysis, the light scattered from the labels can be detected, in the presence of the coating, as an indication of the presence and/or amount of at least one analyte on the sample device, prior to storing the device. The period of storage can vary, with the limit on reproducible repeat detection generally limited by the stability of the coating material selected, in view of the storage conditions selected. Parameters that can significantly affect the practical storage period include extent of exposure of the coating to light (especially ultraviolet light), storage temperature, humidity, exposure of the coating to chemicals that can chemically react with the coating material at a significant rate. In certain embodiments, a storage period can be at least 1, 2, 4, 6, 8, 12, 16, 20, or 24 hours. In preferred embodiments, the storage period is at least one day, one week, 2 weeks, one month, 2 months, 4 months, 6 months, 9 months, one year, five years or even longer.

In certain embodiments, the storing and detecting are performed a plurality of times over a period of time. Where different types of labels or different light scattering particles are used, the different types of labels or light scattering particles can be detected and analyzed at different times, possibly using different instruments. In certain embodiments, especially where multiple samples are present on a device, the labels on different portions of a sample or different samples can be detected and analyzed at different times. Preferably, the light scattered from the particles remains substantially constant (under the same illumination and detection conditions) following storage.

In certain embodiments, the method also includes washing the coated sample device before initial and/or repeat detection. Such washing is useful to remove background light scattering, e.g., from dust particles. The coating protects the light scattering particles from being washed or abraded away. Preferably the wash conditions are physically and chemically mild. Thus, for example, preferably there is no abrasive cleaning, and the wash solution(s) are chemically mild for the particular coating. Preferably the wash solution is an aqueous solution. Such aqueous solution may contain a buffer(s) and/or mild detergent and/or low to moderate ion concentration. Other or alternate compatible components may also be present. Other solvent compatible with the coating may be used instead of water. A compatible solvent (or solution) does not significantly degrade the coating in a manner interfering with repeat or delayed detection. In some cases, a solvent or solution (and accompanying wash conditions) may be selected that dissolves a thin layer of the coating, thereby providing a fresh coating surface. Preferably such dissolved thin layer does not exceed 1, 2, 5,10, or 20% of the coating thickness.

Alternatively, or in addition, the sample device can be re-coated using the same or a chemically compatible different optically transmissive material. Upon completion of the re-coating, the sample device can be reanalyzed. This approach is useful in a variety of situations, for example, where there is accidental scratching or dust accumulation due to improper storage and handling.

One practical result of the present invention is the provision of quantitative RLS calibration standards, enabling normalization of results obtained by different operators, at different times, with different equipment, to obtain absolute quantitative results. This kind of universal calibration and absolute quantitation is not currently possible using fluorescence or other detection reagents or equipment, where only relative signals can be obtained. Physical durability, for example, by coating with a coating composition that solidifies, is an important property to ensure the stability of these calibration standards over time. Thus, in another embodiment, the invention provides sample devices which are designed and manufactured for the purposes of calibration and standardization of results, as well as reagents and apparatus for this purpose.

Many other types of sample device are contemplated for use in the present invention, such as but not limited to forensic sample device, identification sample device, and clinical sample device. Forensic sample device refers to a sample device that has a sample or samples relating to a law enforcement investigation and/or legal proceeding. Thus, for example, the forensic sample device can have sample(s) from a suspect(s) and/or victim(s), or can have crime scene samples. Identification "sample device" refers to a sample device with sample(s) selected to provide identification of an individual organism, preferably a mammal, more preferably a human. For example, the device may be an array providing genotyping information to distinguish the sample source individual from some or all other individuals. Clinical sample device or patient sample device refer to a sample device with samples from one or more individuals selected for medically-related purposes (e.g., clinical or medical research purposes). The sample device and the associated samples are typically selected and configured to diagnose the presence, absence, or status of a disease or condition in the patient, or the susceptibility or resistance to the occurrence or certain courses of development or outcomes of a disease or condition. Alternatively, a patient sample device is configured for research purposes, for example, to provide a comparison of genetic characteristic or gene expression levels between a patient or patients having a disease or condition with one ore more control individuals not having the disease or condition and/or individuals having a different form or severity of the disease or condition. The patient sample use is advantageous in a variety of situations, for example, where a permanent record of an assay result may be desired.

### 5.3 COATING COMPOSITIONS

In various embodiments of the invention, a sample is preserved by being covered or coated with at least one layer of a coating composition that immobilizes and protects the sample, and allows detection of labels on the sample. Preferably, the sample is retained on a solid phase, such as a sample device. Depending on the materials used, the coating composition that covers or coats the sample can become part of the sample device.

A large number of different coating materials can be used in the coating compositions of the present invention. In one embodiment, the coating or coating composition comprises polymeric compounds, such as but not limited to alkyd resins, polyurethanes, acrylics, polyesters, carbohydrate polymers, epoxide polymers, polyvinyl alcohols (PVA), polyvinyl acetates (PVAc), terpenes, and organic-inorganic network materials. Coating materials can also include co-polymers of different materials. These compounds are found commonly in products such as lacquers, varnishes, adhesives, and industrial coatings. Exemplary commercial products that can be used as a coating composition are available under the names Zar Interior High Gloss and Varathane 900 (polyurethanes based on diisocyanate chemistry) RUSTOLEUM™ (clear coat paint), KRYLON™ (clear coat acrylic), DEFT™ lacquer, and PLASCRON™, among others. Also provided are BREAK-THROUGH™ from Midwest Industrial Coatings, Inc., and FICOLL™ from Sigma-Aldrich, preferably modified, as described in section 5.5 below. Photographic lacquers can also be used which can contain cellulose, phthalate esters, and acrylics. Other examples also include biopolymers and other water-soluble materials that cure or dry to form an optically clear coating (e.g. many plasticizers available through large chemical manufacturers/distributors). Further, photocatalytically cured polymers, such as the polymer formulations used in Optics assembly (microscopes, telescopes, etc.) which cure through long wave or short wave UV light reactivity with mercapto-esters, can also be used as coating materials. These materials and compositions can be advantageous in view of the manufacturing, shipping and handling issues associated with many organic-based coatings.

Described below are categories of polymeric compounds that can be used as coating materials in the coating compositions of the invention. Coating compositions comprising these polymeric materials or the polymeric compounds by themselves can be readily tested for suitability by techniques known in the art. The choice of coating material and curing agents (if needed) depends on the application, the process selected, and the properties desired, and are preferably non-toxic. Those of ordinary skill in the art will readily be able to select a preferred material for a particular implementation based on the desired properties described in the next section.

In one embodiment, the polymeric compounds are polyesters, formed by difunctional acids or anhydrides (e.g., fumaric, maleic, isophthalic, terphthalic acids) and difunctional alcohols (e.g, ethylene glycols, propylene glycols). Preferred polyesters include low molecular weight hydroxy-terminated oil free polyesters. In this group of compounds, viscosity is increased in general by increasing the number of functional groups per molecule.

In another embodiment, the polymeric compounds are acrylics where a significant number of the monomers are acrylic or methylacrylic esters, and where other copolymers may include styrene, and vinyl acetate. In an embodiment, the coating comprises an aqueous polymer or an organic polymer.

In yet another embodiment, the polymeric compounds are epoxy resins, including but not limited to glycidyl epoxy (glycidyl-ether, glycidyl-ester and glycidyl-amine), and non-glycidyl epoxy resins. An exemplary epoxy resin is bisphenol A epoxy resin. Typically epoxy resins are cured to form a highly crosslinked, three-dimensional network that is hard, infusible, and rigid. Epoxy resins cure quickly and easily at practically any temperature from 5-150°C depending on the choice of curing agent. A wide variety of curing agent for epoxy resins is available depending on the process and properties required. The commonly used curing agents for epoxies include amines, polyamides, phenolic resins, anhydrides, isocyanates and polymercaptans. The stoichiometry of the epoxy-hardener system also affects the properties of the cured material. Employing different types and amounts of hardener which, tend to control cross-link density vary the structure. Primary and secondary amines are highly reactive with epoxy and are most commonly used. Tertiary amines are generally used as catalysts, commonly known as accelerators for cure reactions. Use of large amount of catalyst achieves faster curing, but usually at the expense of working life, and thermal stability.

In yet another embodiment, the polymeric compounds are polyurethanes which are typically formed by cross-linking hydroxy-functional polyesters and acrylic resins with aliphatic or aromatic isocynates. The reaction proceeds relatively rapidly at ambient temperatures. Examples of polyurethanes used in lacquers and varnishes are polymers of toulene diisocyante, hexamethylene diisocyante and tetramethylxylidene diisocyante. Some varnish products for the do-it-yourself markets contain urethane oils (also known as urethane alkyds or uralyds) which are formed by a diisocyante with partial glycol esters of drying oils. Such compositions generally lack unreacted isocyanates and are preferred for its low toxicity.

For use in the present invention, the above described polymeric compound(s) are present in a coating composition individually or as a mixture as in many commercial products.

Solvents that can be used in conjunction with the coating composition to alter its various properties, such as viscosity, wetting and volatility, include but are not limited to acetone, toluene, methanol, methylene chloride, n-methyl pyrrolidone, 2-butanone, 2-butoxyethanol, xylenes, and di-basic esters. Such solvents have been used in the development of solvent-based polymer coatings for home furnishings and home-building. While the components of such formulations are well known, the relative amounts of the components in the formulations are determined empirically based on the properties of the surface to be coated by methods known in the art.

## 5.4 COATING PROCESS

Coating of sample devices can be performed in a variety of ways, including without limitation spraying, dipping, and pouring methods, and sputter deposition, evaporation, plasma-enhanced deposition and masking techniques. One of ordinary skill in the art of applying coatings will recognize that selection of a suitable coating method will depend on the specific coating selected, the character of the resulting finished coating needed, properties of the surface, convenience, cost, and other process factors. In some embodiments, the coating composition requires curing in order to form the final coating. Curing can be accomplished by exposure to physical agents, such as heat and/or light, or chemical agents including air, vapor or volatile agents, and gaseous curing agents. In a specific embodiment, after the coating composition is applied, the curing results in a solid or permanent coating.

As is commonly understood in the field of application of thin coatings, spraying may be airless, involving atomization of the fluid as it flows under high pressure from a spray nozzle. Other spray systems utilize a stream of gas (usually air) under pressures of about 30-80 psi to propel and atomize the coating fluid. Spray application may be suitable where the flow characteristics of the coating after application allow formation of a sufficiently smooth and defect free surface to avoid difficulties with light scattering from surface imperfections. Additionally, spray methods are more likely to be suitable in cases where overspray is not a significant problem, and thus is more likely to be applied in cases where large areas are to be coated at the same time.

A dipping procedure typically involves dipping a sample device in a volume of coating composition in fluid phase sufficient to immerse at least the portion of the device surface having attached label or that is otherwise desired to be coated. A dip and dry system can function independent of extensive additional equipment and manipulation. In contrast, chemical and physical methods of inducing crosslinking can be less robust, and may require the use of hazardous materials, and/or greater hands-on manipulation. Typically the device is allowed to drain for a period of time to remove excess fluid coating before the coating solidifies. The device may be allowed to dry or harden in a vertical or inclined draining position, or may be placed in a generally horizontal position to minimize strain and irregularities in the coating as it solidifies. Spinning, e.g., in a low-speed centrifuge can also be used to remove excess coating solution.

Pouring typically involves placing a sample device in a generally horizontal position and pouring the coating composition in fluid phase on the upper horizontal surface. The device may remain in the horizontal position while the coating solidifies, or may be inclined to facilitate draining. As indicated in connection with dipping, spinning can also be used.

A coating which introduces light scatter will increase background noise and reduce sensitivity of light detection and is thus not desirable. The solidified coating is preferably colorless. However, this may not be critical if coating layer is thin where the contribution of color may be minimal and/or where the color can be predicted and accounted for in downstream data quantitation where necessary. The curing method preferably does not involve multiple curing agents, extraordinary manipulations or equipment. Curing times should allow enough time for physical handling after a coating composition is applied but not requiring more than 30 minutes, 1 hour, 3 hours or 6 hours. Thirty (30) minutes to one (1) hour is an example of a reasonable cure time for many applications. Signal strength and resolution before and after coating can be compared in order to refine the process.

Persons familiar with coating materials and processes will recognize many different variations in coating and curing methods that can be used appropriately with specific coating materials.

## 5.5 COATING DEVELOPMENT

As described above, any of a number of different types of coating materials can be used in the present invention depending on the properties required for a particular application. In the process of developing a coating however, there are many practical concerns to be addressed, which will depend on the type of sample device being preserved. There are several practical considerations for identifying or creating coating materials suitable for use in the invention. The physical and optical properties of candidate coating materials, such as viscosity, toxicity, refractive index, etc., will need to be considered in making a selection. Additionally, if one or more of the properties of an available coating material are undesirable, they may be modified by methods described hereinbelow to provide a more desirable material.

The viscosity of the coating material is one of the properties that needs to be considered based on the type of sample device being preserved. In some applications, it might be preferable that the coating material have a low viscosity, if appreciable flow characteristics are desired. An example could be an application where the coating material preferably incorporates with or permeates the sample device. Additionally, low viscosity materials also permit further purification through simple filtration, gravity, etc. if necessary. In some applications, a higher viscosity material might be preferable, e.g., if the coating material is expected to provide some amount of structural support to the sample device. In addition, it may be both be desirable and advantageous to modify a solvent system with a compound (e.g. an oil, or, alcohol) that may also increase the refractive index of the finished coating.

In order to minimize costs associated with shipping and disposal, it may be preferable to choose nontoxic and/or nonflammable materials, which also reduces risk to potential users/handlers. In this regards, it might also be preferable that they are soluble in or compatible with water.

The desired optical properties of the resulting coating will also affect the choice of coating materials. The refractive index of the coated sample device may affect the intensity of any light emitted from labels on the sample device. Additionally, it should also be considered that the refractive index of the coating material changes as a result of the curing/drying process. For example, the choice of a high refractive index material can increase the intensity of the light emitted from a sample device, if the sample device comprises resonance light scattering labels. The colorlessness of candidate coating materials can be an indicator that they will not bias the reflected, refracted or scattered light. Also, visible properties such as low haze or high clarity can also indicate that the coating material will contribute only a minimal scatter background relative to the sample device. If the coating material is found to be too hazy, then preferably a first approach could be to try to reduce the solids content of the candidate material by dilution with a compatible solvent. A second approach is to apply or substitute solvents which, when reacted with the reagent polymer, prevents or slows down crosslinking, or causes the polymer not to crosslink as extensively. In the exemplary case of polyurethane a compatible solvent is a low molecular weight ketone, such as 2-butanone. In the exemplary case of Ficoll/polysucrose or polyvinylalcohol, the solids would simply be reduced in the composition with water to a compatible range. When substituting alternate solvent systems such as ethanol:water or dimethylsulfoxide:water, increased particulates and haze may result, respectively, thus illustrating the utility of the second approach.

The properties of adhesion and/or wetting can vary according to the coating material applied. Ideally, the optical coating material adheres to a wide variety of surfaces under standard temperature and pressure. However, some coatings can exhibit poor adhesion to substrates. In most organic solvent systems this is typically not problematic as many such solvents have tremendous wetting capacity. However, in aqueous systems each polymer must be tested for adhesion and wetting, then modified if necessary. In the exemplary case of polysucrose, which exhibits a poor wetting capacity on some chemically altered glass surfaces, addition of an alcohol will confer greater wetting capacity prior to volatilizing from the surface. Additionally the viscoelastic properties of polymers such as polysucrose and polyvinylalcohol can be modified via hydrogen bonding with mediators such as borate. Addition of borate typically increases the elasticity of the polymers with a high propensity to form hydrogen bonds (e.g. Polysucrose/Ficoll, PVOH).

A coating material that dries or cures to a smooth, high gloss finish may minimize the retention of aerosol debris on the slide surface and provides a smooth, polished, transparent optical coating-air interface. A pitted and/or uneven, inconsistent surface may reflect a greater amount of incident light. The drying and/or curing time can be a factor in the quality of the resulting finish.

Optical coating application may preferably require a short drying time. If the dry time of a coating material is too long, it can be decreased through either (1) modifying the volatility of the solvent system or (2) reducing the viscosity, resulting in a thinner curing layer post application. In the case of organic solvents, typically, a more volatile component of the solvent system could be given a greater partition to achieve a shorter dry time. As 2-butanone is compatible with many organic solvent systems it is the preferred choice in a polyurethane-based system, although, many other options exist and should be tested empirically for greatest compatibility. In an aqueous system such as that with polyvinylalcohol or ficoll, reducing solids often results in lower viscosities leaving thinner drying layers.

Alternatively, other applications might require a longer drying time, e.g., if futher manipulation of the sample device is desired in the presence of the coating material. Organic solvents, and some lacquer/urethane and acrylic based systems are examples of coating materials where drying may occur in a matter of minutes. Under certain conditions frosting (haze imparted on the surface resulting from moisture in the atmosphere condensing on the cooling surface of applied polymer) may result from a formulation that dries very quickly. An example of this can be seen when certain lacquers are applied to substrate-bound nitrocellulose for purposes of clarifying the nitrocellulose and preserving it in one step. The most direct treatment for frosting in this case is to add a partition of 2-butoxyethanol to the original composition to retard the drying process. In this application, the lower volatility of 2-butoxyethanol stabilizes the drying process by conferring its properties on the solvent system as a whole. In some cases, nearly 50% of the solvent formulation has been partitioned for 2-butoxyethanol addition.

### 5.5.1 Low background scattering

In general, it is beneficial to select a material that provides an optically clear coating with low non-specific light scatter. Such non-specific light scatter can arise, for example, from inhomogeneities in the material, including, for example, contaminant particulate matter, solidified material with different refractive index, and bubbles. As a result, a coating material is preferably selected that does not contribute significant background scatter. Further, the handling of the material and the coating process should be done to minimize introduction of scattering materials. Thus, for example, the material should be protected from dust and other airborne particles, and handled in a manner to avoid creation of bubbles. However, if particles or bubbles are present, such can generally be removed by filtration and de-gassing respectively.

Following initial coating, the coated sample device should be handled in a manner to avoid introduction of non-specific light scatter. In general, it is beneficial to have a surface on the solidified coating that is as free as possible from defects. Such defects can include foreign material and/or surface irregularities. For example, during solidification, the coating should be protected from particles that could deposit on the coating surface. Likewise, the solidification, curing or plasticizing should be carried out in a manner that does not introduce surface irregularities, e.g., contacting the surface before the coating is fully solid or permitting flow of partially solidified material (*i.e.*, rippling). In this regard, agents that are fairly nonviscous and exhibit self-leveling properties are particularly useful.

As used herein, the term "solidifying" refers to a transition from a liquid to a solid state or phase, where the term "solid" has its common meaning, indicating that the material has sufficient coherence of form to distinguish from liquids and gases. In many instances, the process of curing comprises solidifying of a coating composition. As used herein, the term "solid" includes gel. However, preferably, the solid material has sufficient coherence of form that there is no fluid flow visible to the human eye when held in any position for 10 hr for amounts and shapes of a material as used in the present invention. Highly preferably the material shows no deformation visible to the human eye when subjected to moderate pressure with a human finger for 5 seconds. Solidifying may involve various processes, e.g., drying, cross-linking, polymerization, and/or other reactions that reduce the freedom of movement of component molecules in a solidified material sufficiently to result in a solid. Solidifying differs from a situation in which a suspension or colloid of solid particles in a liquid or gas are formed. In such suspensions or colloids, the bulk solvent remains liquid or gas and only the colloidal particles are solid material, while in the present solidified material the chemical and physical interactions resulting in the solid occur through the solidified coating and are not restricted to colloid particle scale.

As used herein, the term "solution" refers to a material with a predominantly liquid bulk property. Thus, the term includes true solutions, as well as suspensions, liquid medium colloids, and emulsions.

### 5.5.2 <u>Clarity</u>

Coatings useful in this invention should allow for light transmission in a largely unobstructed, non-scattering manner. Thus, opaque coatings generally cannot be used. In addition, particularly for use with RLS labels, as indicated above, the coating should not contribute significantly to background light scattering. Thus, translucent coatings are not preferred, even though they permit the passage of substantial light. It is highly preferred that the coating lack any visible cloudiness or similar characteristics.

The terms "clear", "optically clear", "transmissive", "transparent", and "transparency" refer to the ability of a material or medium, e.g., a coating material and/or support material, to transmit light sufficiently and sufficiently free from cloudiness and the like that images are readily discernable through the material. In the case of materials that are in the light path for illumination or detection for a sample, the term indicates that, in the amounts used in a particular case, the material does not substantially interfere with the passage of light through the material to an extent to prevent reproducible repeat detection of scattered light from light scattering particle labels associated with the sample. Such interference may include, for example, absorption, reflection, and/or scattering by the material. Highly preferably, in the amounts used in the present invention, an optically clear material does not reduce the intensity of light passed through the material by more than 30, more preferably by no more than 20%, still more preferably by no more than 10%, and most preferably by no more than 5%, 4%, 3%, 2% or 1 %. It is understood, however, that these terms do not necessarily mean that the material is completely colorless. However, the amount of color and/or the wavelengths of light not passing through the material are such that it does not prevent use of the coating in the assay. For example, even a relatively highly colored material may be used if the coating is sufficiently thin that the fraction of light reflected or absorbed is small enough to not preclude effectively carrying out the assay, and may be small enough to be negligible. Likewise, the wavelengths of light reflected or absorbed may be such that it does not prevent effective illumination and detection of the labels.

However, it is only important that the coating is transparent with respect to relevant wavelengths of light. For example, in particular applications, a coating may highly absorb ultraviolet, or near ultraviolet wavelengths without interfering with performance of an assay, due to the light wavelengths detected. Similarly, a material may significantly absorb infrared wavelenghts, but still not interfere with performance of an assay. Preferably, the coating should not prevent use of visible wavelengths of light, especially in the 400 to 700 nm wavelength range, or at least 450-700 nm range.

### 5.5.3 Durability - chemical & physical

In many applications, it is highly beneficial if the coating is physically and/or chemically durable. If a sample device is to be read immediately and not stored for later reading, these characteristics are of less importance, a softer and/or less chemically resistant coating may well be acceptable. However, in general, a hard coating is preferred. Resistance to chemicals that may be encountered is also advantageous.

With respect to chemical resistance, in particular embodiments the optical properties of the coating are unaffected by a brief rinse with water and preferably are unaffected by exposure to water at room temperature for up to 1 hour, preferably up to one day, or longer. Preferably the coating is also similarly resistant to solutions with which a coated sample device is likely to come in contact, for example, one or more of the following: common buffers used in biological laboratory practice, microscope immersion oil, detergent solutions, ethanol, propanol, and the like, as well as mixtures of ethanol and/or propanol and water.

With respect to physical durability, the most important characteristics are scratch resistance and resistance to embedding of foreign particles. While the Moh's scale is usually used in connection with minerals, applying it to coatings, a coating for use in this invention preferably is at least 1.5 more preferably at least 2, 2.5, 3 or 3.5 on that scale, with higher values being more preferred. In terms of exemplary comparisons, preferably a solidified coating has a hardness and scratch resistance greater than the average for commercial outdoor application alkyd enamel paints applied according to manufacturer recommendations and allowed to dry for one week at 23 °C with 50% humidity.

### 5.5.4 Thickness

In certain embodiments of the invention, one or more layers of optically transmissive material is used to coat the sample and/or sample device. Each layer may have a different thickness. The thickness of the coating is that which allows effective illumination and detection of labels on the sample. In embodiments where multiple layers of coatings are used, the thickness of the layers as a whole should not impair effective illumination and detection. For sample devices having small or densely packed features and for detection of single particles, it is highly preferred that the coating does not distort the signal or image (or degrade signal strength and/or resolution) to an extent below the level needed in a particular application, e.g., to be able to distinguish adjacent microarray features. Typical coating thicknesses will be in the range of 1 micrometer to 1 mm inclusive, preferably in the range 1 micrometer to 0.1 mm, or 0.02 mm to 0.1 mm.

### 5.5.5 Viscosity Modification

While any of a number of suitable coating materials can be used, the invention provides that modifying the viscosity of coating materials can be beneficial. In one embodiment, the invention provides that dilution of coating material comprisng polyurethanes, many organic solvents, for example lacquers and other clear coat finishes with highly volatile ketone-based solvents have the effect of reducing viscosity, and reducing cure time of the original materials and coating formulation, thereby rendering the materials, more advantageous as coating materials for use in the methods of the invention. These features can provide the following benefits: user handling time is decreased (e.g., 3-4 hour cure time on original form, ~1 hr on the diluted form); the resulting layer (or tegument) is thinner (viscosity reduction allows more complete draining of excess material); background levels are reduced because the concentrated form may have higher levels of particulate materials; and no lip or formation of hardened excess material present at the lower end or edge of the substrate with inclined positional drying is apparent on the solid phase after drying. Examples of such ketone-based solvents include but are not limited to 2-butanone, and acetone.

In another embodiment, the converse may be desirable. That is, decreasing volatility of liquid coating materials prior to application to a sample or sample device can be beneficial. In one embodiment, the invention provides the addition of 2-butoxyethanol to increase the cure time by decreasing the overall clear-coat solvent volatility. In another embodiment, a class of aromatic compounds, such as benzaldehyde and toluene, that contain an aromatic ring, generally a hydrocarbyl ring, and most often a phenyl ring, can be used to achieve the same effect. The increased cure time is desirable, for example, in the case where short cure times may introduce frost upon the coating; a phenomenon attributed to moisture deposition upon the coating surface during the cure process.

The addition of preferably 2-butoxyethanol but also others of this class of aromatic compounds may also improve the flow character of the coating, especially on glass and compatible plastics. For certain fast curing liquid coating materials, the flow of liquid is rapidly "frozen" to a solid phase, which may introduce striations on the coating surface. The result is an imperfect surface, potentially introducing additional light scatter.

The same approaches may also be applied to other coating materials using chemically compatible solvents of higher or lower volatility. Such compatible solvents can readily be selected based on the known chemistry of a particular coating and/or by empirically testing and confirming compatibility.

## 5.6 <u>MEMBRANE PRESERVATION</u>

In another embodiment, the present invention provides for the preservation of a membrane comprising a sample and/or one or more labels present in and/or on the membrane. In preferred embodiments the membrane is a sample device, and a coating composition is applied to the sample device so as to form a coating to preserve the sample device.

As used herein, "membrane" refers to a thin, flexible material, that can be impermeable or microporous, and preferably synthetic material. Preferably pores or channels if present in the membrane (also known as membrane filter) are no larger than 20 $\mu$m, more preferably no larger than 10, 5, 2,1, 0.5, 0.2 or 0.1 $\mu$m, or in a range specified by any two of these specified endpoints. A membrane can be, for example, a uniform sheet of material with essentially uniform composition and properties, e.g., a film, woven or matted fibrous material. Examples of commonly used materials include nylon, nitrocellulose, polyvinylidene fluoride (PVDF), and cellulose. The membrane can have any of a range of surface areas typically determined by the intended application, e.g., the size and number of features in an array. Thus, in particular embodiments, the membrane sample device has an area of less than 1 in$^2$, 2 in$^2$, 4 in$^2$, or 10 in$^2$, though larger membranes can also be used. Preferably, the membrane is colorless.

Membranes supported on or attached to solid supports present a set of technical issues for light scattering particle labels. For example, incident white light can be scattered by unclear substrates, non-specific particulates, molecules and substrate surface irregularity. Particularly relevant to membranes bound to solid supports is the lack of clarity. Accordingly, these membranes should be rendered substantially clear optically in order to obtain a robust and specific signal from light scattering particle labels on the membrane.

The use of liquid materials to clarify membranes has been described in Brooks, U.S. Patent 6,165,798, which is incorporated by reference herein in its entirety. The Brooks patent mentions the use of polyethylene glycols (PEGs), polyvinlypyrrolidone (PVP), polyethyleimine (PEI)(refractive index = 1.52-1.53), benzyl alcohol (refractive index = 1.538), and PEI plus water in addition to other agents that clear or dissolve the membrane. Other liquid clarifying materials are Type A immersion oil and benzenemethanol (refractive index = 1.539). The drawback to these approaches is that they are neither "user friendly", nor compatible with routine instrument operation due to their inherent need for "wet" chemistry; the membrane must remain wet during analysis. These materials remain in liquid form while the membrane is clarified or dissolved, and do not preserve the membrane for repeated and postponed analysis. Brooks also disclosed the use of PEG with a molecular weight of 1000 with nitrocellulose, that is solid which is incorporated into the membrane but which clears the membrane only when it is liquefied. Accordingly, the use of polyethylene glycols, polyvinlypyrrolidone, and polyethyleimine in the coating compositions of the invention are less preferred.

The present inventors discovered a method and a class of coating composition which simultaneously transparify membranes, such as cellulose nitrate membranes, while producing a durable tegument around the membrane thereby preserving it. In this process, the membrane is transparified by the coating composition and remains clear while the coating composition solidifies to form the coating. Thus, the advantages over Brooks such as reduced background, increased signal, dry operation and preservation of the membrane for future analysis are realized. In addition to cellulose nitrate membranes, this method and class of reagents can be applied to many types of membranes used in biotechnology, but not limited to membranes made of cellulose nitrate, such as nylon and polyvinyl difluoride (PVDF).

In one specific embodiment, the invention provides a one-step method for transparifying and preserving a membrane, by treating the membrane with a solidifying, non-dissolving, optically transmissive coating composition. The term "non-dissolving" indicates that the solution does not dissolve the membrane matrix such that the membrane remains substantially intact. The term "transparifying" refers to substantially reducing the light scattered by the membrane under particular illumination conditions, e.g., by contacting the membrane with a fluid that reduces light scatter from the membrane. Typically and preferably the process increases the transparency of the membrane by at least 10%, 20%, 30%, 50%, 75%, 90% and 98% compared to a membrane without treatment. Preferably the fluid is an optically clear fluid. While optically transmissive solidifying solutions can be used for the fluid, in other embodiments of the various aspects optically transmissive non-solidifying solutions can likewise be used. Preferably, the membrane also comprises light scattering particle labels. Preferably, the coating composition does not dissolve or interfere with either the sample or the labels.

In preferred embodiments, the membrane is associated with an optically clear or transmissive solid phase support. As used in connection with membranes and solid phase supports, the term "associated with" refers to any manner of interaction that retains a membrane adjacent to a solid phase. Thus, the term includes, for example, attached to, resting on, bonded to, clipped to, and supported by the solid phase support. Preferably the solid phase support is glass or plastic. For example, in particular embodiments, the membrane is attached to or supported by a physical structure, such as a frame, or bonded to a slide. The term "attached" refers to physical retention of the membrane by the support with sufficient strength to retain the membrane under normal handling in any position. This is distinguished from "supported", which refers to retention of the membrane on the solid support under the force of gravity, but which may not retain the membrane in position in all orientations. Support does not involve physical clamping or chemical bonding, or other similar strong chemical or physical means. The term "bonded" indicates that the involvement of chemical bond interactions and/or the use of an adhesive.

There are substantial benefits of a coating composition capable of both transparifying and preserving membranes associated with solid supports, and preferably ones capable of achieving this with a single treatment step. These include, without limitation:

1. Membrane transparification minimizes non-specific scatter introduced by the substrate on which the immobilized labels have been attached.

2. The preservation process has the end effect of preserving the specifically attached light scattering particles in a material, that yields greater light scattering particle signal intensities, relative to air.

3. The preservation process is also capable of dissolving and transparifying non-specific scattering debris that are inseparable from the solid support by routine processing.

4. The preservation preferably results in a smooth, regular surface.

5. The preservation process both protects and preserves the membrane, as well the specific signals retained on the membrane, indefinitely. As previously described, RLS particles are not subject to compromised signal strength over time. The marriage of the signal integrity of the RLS particles with preservation lends a tremendous advantage over other light detection technologies in the frequency and duration of time over which an RLS particle signal can be read.

6. The preserved sample device can be cleansed with mild solvents to remove unwanted accumulated debris and oil any time after the preservation/transparifying coating has fully cured.

In embodiments where a membrane is bonded to a slide or other support, preferably the bonding uses an adhesive which can be in various forms, for example, sheet, liquid, gel, aerosol and semi-liquid. Preferably, but not necessarily, the adhesive is optically transmissive or becomes optically transmissive following bonding. Such optical clarity is especially useful when illumination and detection are on opposite sides of the support, but can also be beneficial in other configurations, e.g., to reduce non-specific scattered light. In other embodiments, the bonding involves direct chemical interaction between the membrane and the support, e.g., a functionalized surface of the support.

While the use of refractive index matching materials can achieve membrane transparency, the invention encompasses using solvents/solutions with lower refractive index in combination with chemical modification to substantially reduce cross-linking in a membrane. The inventors observed that 100% ethanol can render a nitrocellulose membrane nearly transparent and believed that simple reduction or modification of cross-linking structure can facilitate transparification. Apparently, chemical modification of a membrane by a transparifying agent after an assay has been completed does not affect the result. In addition, disassembly of the membrane's extensive architecture without dissolving the membrane may help to reduce residual haze produced by networks of cross-linked polymer. This residual haze can easily be visualized with the aid of a Tyndall beam. Thus, in certain embodiments, the coating material also includes an agent or agents that chemically modify the membrane, e.g., by reducing crosslinking in the membrane, though without dissolution of the membrane as described in Brooks et al., U.S. Patent 6,165,798.

In a particular embodiment of the invention, the coating composition comprises terpenes, preferably monoterpenes, and most preferably beta-pinene. Terpenes are widespread in nature, mainly in plants as constituents of essential oils. Many terpenes are hydrocarbons, but oxygen-containing compounds such as alcohols, aldehydes or ketones (terpenoids) are also included. The building block for this group of compounds is isoprene, $CH_2=C(CH_3)-CH=CH_2$. Terpene hydrocarbons therefore have molecular formulas $(C_5H_8)_n$ wherein they are classified according to the number of isoprene units: monoterpenes, n=2; sesquiterpenes, n=3; diterpenes, n=4; triterpenes, n=6; and tetraterpenes; n=8.

A coating composition comprising an organic solvent (or a mixture of organic solvents) with at least a terpene can be used in the simultaneous transparification and preservation process of the invention. In a preferred embodiment, beta-pinene, which is a bicyclic terpene $(C_{10}H_{16})$ is included in the coating composition. Beta-pinene provides the advantageous optical characteristic of minimal light absorbance, reflection, and scattering at shorter wavelengths (e.g., 220 - 450nm), as compared with many other systems, e.g., lacquer based systems. Exemplary organic solvents used to dissolve beta-pinene pellets include but are not limited to toluene and xylene. The formulation of the final agent in part depends on the thickness of the membrane layer. Additionally, coating compositions comprising beta-pinene are preferred for very thin membrane layers associated with a solid phase such as a glass, plastic slide or film.

The use of beta-pinene in a coatingcomposition is not restricted to a system comprising membranes. Beta-pinene can be used in a coating composition to preserve other sample devices comprising glass or plastic. In such applications the solvent(s) used would comprise those common to many lacquer thinning systems well known in the art. This modification is preferable to achieve liquid wetting and flow properties compatible with glass and plastics. Toluene or xylenes are less preferred as they exhibit poor flow properties with respect to coating glass and plastic surfaces.

An exemplary coating composition comprises beta-pinene pellets dissolved in a selected organic solvent to a 5%,10%, 15%, 20%, 25% and 30% final concentration. The desired concentration depends on such factors as the membrane thickness. The coating composition is preferably sealed in a container to prevent evaporation. The coating composition is applied to a membrane or a sample device comprising a membrane. In one embodiment, the membrane or sample device is dipped in the coating composition one or more times, preferably as many times as necessary to remove all air bubble or particulate matter. A thin coating is preferable. The coated membrane or sample device is allowed to cure for several minutes, and preferably 10 to 15 minutes. In other embodiments, the coating composition comprising beta-pinene can also be used for preserving non-membrane sample device, such as glass and plastic microscope slides.

In another embodiment, the coating composition comprises clear wood finishing lacquer. A wood finishing lacquer (e.g. Parks Clear Lacquer™ or Deft Lacquer™) can be mixed with an organic solvent or a mixture of organic solvents for use in the simultaneous transparification and preservation sample device. The formulation of the coating in part depends on the thickness of the membrane. In preferred embodiments, methyl ethyl ketone or ethylene glycol monobutyl ether are used as solvents for the preparation of a coating composition comprising wood finishing lacquer. A coating composition comprising wood finishing lacquer is preferred for nitrocellulose membranes.

Depending on the thickness of the membrane, a coating composition comprises at least 20%, 25%, 30%, 45%, 50%, 65 % wood finishing lacquer mixed in a selected organic solvent (or mixture of organic solvents). The wood finishing lacquer is allowed to dilute into the organic solvent (or mixture) and is incubated preferably at room temperature to form the coating composition. Preferably the coating composition is sealed in a container to prevent evaporation. The membrane is exposed to the coating composition by, e.g., dipping the sample device one or more times in the coating composition, preferably as many times as is necessary to remove air bubbles or particulate matter. A thin coating is preferable. The coated sample device is allowed to cure for several minutes, and preferably 30 to 45 minutes.

The optical clarity after preservation is important for signal quantitation using RLS detection. Properties of the membranes such as the thickness, and the adhesive material used, can influence preservation performance. The pore size of membrane layer on a transparent support may also influence the preservation agent formulation and procedure for optimal performance and detection sensitivity.

Other reagents or assay conditions in membrane slide processing besides the preserving step can also influence RLS detection performance. For RLS detection on membrane arrays for example, the pre-hybridization, hybridization and post-hybridization wash solutions are formulated and optimized with the membrane and preserving agent to prevent "frosting" within the preserving agent coating that appears as the formation of an opaque layer. Other considerations for detection performance include hybridization temperature, time and stability of the membrane (either cast or laminated) on the transparent support.

Techniques known in the art can be applied to optimize the procedure for preserving a given type of membrane or sample device and experimental protocol for the best performance of RLS particles for analyte detection.

### 5.7 STORAGE

For sample devices that are to be stored for later analysis (initial or repeat), it is highly preferable to store the device in a manner that avoids creation of defects that can degrade the sample and results. Such defects can form in various ways, for example, photo-damage, physical damage, chemical damage, and presence of foreign material (e.g., dust) on the surface.

Many types of coating materials will be subj ect to photo-damage. Such damage is especially likely to be caused by ultraviolet (UV) light due to the high energy of such light. Such photo damage can include introduction of color to and physical degradation of the coating, especially the surface, with concomitant increase in background light scattering and reduction in reproducibility of illumination of the labels and detection of specific signals.

Such photo damage can be reduced to low levels by storing the coated sample device in "dark conditions", which refers to dim light as perceived by humans with normal vision, but, unless otherwise specified, does not require complete darkness. Recognizing that UV light is particularly significant for degradation of materials due to photo-damage and UV-induced chemical changes, dark conditions refer to a level of ultraviolet light that is no greater than 10% of the intensity produced by a standard 40 watt fluorescent light bulb designed for work or residential area illumination measured at a distance of 2 meters and averaged across the UV spectrum. More preferably, the dark conditions UV intensity is no more than 5%, 2%, 1%, 0.5%, 0.2%, 0.1%, or even less as compared to the fluorescent light bulb intensity as indicated. Likewise, preferably other wavelengths are reduced to the same intensity % range as the UV. Such dark conditions can be interrupted, excluding brief periods when a storage container or other space may be opened or accessed, e.g., for introduction or removal of a sample device.

In preferred embodiments, the method also involves storing the sample device, preferably under dark conditions. Such dark conditions, for example, storage of a sample in a slide box, are commonly recognized to reduce or eliminate light-induced degradation of materials, especially UV light induced degradation.

In preferred embodiments, the method also involves storing the sample device for an extended period of time, preferably without significant degradation of the labeled sample to generate a detectable light scattering signal. Such degradation can occur, for example, through bleaching, quenching, decay, or chemical degradation of the label, and/or

EP 2 051 074 A1

through degradation the coating. Degradation of the coating can, for example, result in increased cloudiness or even opacity, increased coloration, and/or increased light scattering. In particular embodiments, the preserved sample device is stored for a period of at least 1, 2, 4, 6, 8, 10, 14, 21, or 28 days. In further embodiments, the preserved sample device is stored for at least one week, 1, 2, 4, 6, 8, 10, or 12 months, or even more.

In addition to photo-damage, coated slides can be subjected to physical damage. That is, the coating can be damaged by physical contact, thereby creating surface defects that can contribute to an increase in non-specific background and/or reduced lifetime for the coating. Such physical damage can include, for example, abrasions, cuts, and embedded particles. Moderate care in handling will avoid most such damage, e.g., handling sample devices by the edges, avoiding contacting the surface with sharp or abrasive surfaces, and using care in cleaning dust or other particles from the surface.

Additionally, the coating surface may be damaged by chemicals. Such chemicals, may, for example, be in wash solutions and/or fumes. In many laboratory settings, fumes from a variety of different chemicals may be present. Depending on the chemical characteristics of the coating, the fumes may react with the coating, damaging the surface. Thus, in general, it is desirable to avoid contact with such fumes that will react with a particular coating, especially for extended periods of time. Likewise, if they are to be used, wash solutions should be selected that do not significantly react with the coating, either by chemically modifying the coating, or by dissolving the coating. (However, a slight dissolution can be advantageous as it can provide a new surface, removing or reducing slight surface defects.)

It is desirable to minimize deposition of foreign materials such as dust on a coating during storage or analysis. However, in the event dust or other materials are found on the surface, the solidified coating can be washed and/or cleaned with a gas stream (e.g., air or nitrogen). Such wash solution and/or gas should itself be essentially free of foreign mediums that would deposit on the coating surface. In addition, as indicated above, the wash solution and/or gas should be selected that are chemically compatible with the coating medium. Further, the surface cleaning should be conducted in a manner to avoid physical damage. For example, washing should be done to avoid abrasion damage to the surface, e.g., by using a gentle to moderate liquid stream without wiping or scrubbing. Physical damage can also be avoided by selection of a hard coating in preference to a softer coating.

In addition, in some cases, samples that have been preserved and experienced physical damage due to surface scratches or other defects or contamination, the sample can often be recovered to its original quality by simply retreating the sample with the same preserving agent, or a different, chemically compatible preserving agent. This aspect adds to the permanency of the sample preservation using the present invention. Semple devices with aqueous coatings can be stored in sealable containers or with non-dust producing dessicants, e.g., dessicant enclosed in a plastic housing to limit exposure to humidity or moisture from the air.

One of ordinary skill in the art will be familiar with the factors relevant to avoiding coating damage. Sensitivity or resistance of a specific coating to damage from a particular condition can also be determined empirically by exposure and inspection, e.g., under high magnification and/or in assay or assay simulating conditions.

## 5.8 PRESERVATION KITS

In another embodiment, the invention provides a kit that provides materials and instructions for carrying out the methods of the invention, including but not limited to performing analyte assays, preserving sample devices, storing, retrieving, analyzing and reusing the devices. In a preferred embodiment, the kit comprises a coating composition and analyte-binding light scattering particle labels in separate vials.

Typically the kit will be packaged in a single container. The coating composition is highly preferably packaged under conditions such that the solution will not solidify or become cured for a period of at least one week, more preferably at least one month, still more preferably at least two months, and most preferably at least 12 months or more. Alternatively, the kit can comprise a concentrated form of the coating composition (*e.g.*, 2x, 5x, 10x concentration) and a diluent (or solvent) in separate vials. The diluent is used to prepare a coating composition of a desired concentration. In a case where the coating material is a solid or powder, both the solid or powder and a diluent are provided. In an embodiment, the kit can contain a set of instructions for preparation of the coating composition, a procedure for coating the sample or sample device, and/or recommendations for storage of the preserved sample device.

In another embodiment, the kit can comprise a coating composition, and a curing agent specific for the coating composition. In yet another embodiment, the kit can comprise a coating composition, and a removal agent that can remove a coating or coating composition that is present on a sample or sample device; preferably, the removal agent does not distort or damage the sample. In yet another embodiment, a kit can comprise a coating composition and any one or more of the following: a diluent, a curing agent, and a removal agent.

The light scattering particle labels can be supplied in the kit in various forms, depending on the intended application, e.g., for use directly with assays, or for use in constructing custom assays. Thus, in certain embodiments, the light scattering particle labels have a moiety or moieties that bind to analyte under binding conditions. Such moieties include without limitation, specific oligonucleotides, antibodies and antibody fragments, specific antigens, haptens, biotin, avidin and streptavidin, as well as other members of specific binding pairs and other molecules that provide specific binding. The binding to an analyte can be direct or indirect. Likewise in certain embodiments, the light scattering particle labels have moieties that bind to analyte binding molecules under binding conditions. For example, the particle can have on its surface a moiety for attaching a nucleic acid or a protein, or other molecule that can provide direct or indirect analyte binding.

The kit can also include at least one sample device, e.g., at least 1, 2, 4, 6, 8,10, or more sample devices. As with aspects described above, such sample devices include without limitation arrays, microarrays, array chips, slides, microtiter plates, and membranes.

The kit can also include an instrument for detecting the labels on a sample device. Depending on the type of labels used, the instrument can be very simple and portable. Such instruments can include a light source, a place to hold a sample device, and means for detection or assisting detection, such as light collection optics for direct viewing, light sensors and photographic equipment. Typically detection involves the application of a linked computer and associated software to help interpret, quantify and/or document experimental data.

## 5.9 GENERATION AND USE OF A CALIBRATION DEVICE

The ability to preserve a sample device, and to store it as desired, without significant degradation of the detectable signal provides advantages in a variety of situations. For example, such preservation and the ability to store sample devices allows repeat reading of the assay results for an experiment, as well as delayed reading of assay results. This allows the sample device and/or assay results to be used over a period of time and/or between different laboratories while still obtaining comparative results. Such comparative results can be obtained even when different instruments are used, by calibrating the instruments or results with a standard "calibration" device (such as a calibration slide).

In an embodiment of the invention calibration device comprising RLS particles is provided which can be used to calibrate various parameters such as exposure time, camera gain, resolution and the like generally associated with RLS detection. Use of calibration slides or other calibration devices is beneficial, e.g., to assist in cross-instrument, cross-experiment, and/or cross-laboratory comparisons of assay results. In a preferred embodiment, such a calibration device would be a glass microscope slide upon which RLS particles of defined optical properties are deposited at predetermined particle surface densities measured in particles/square micrometer. Although other substrates or assay formats, for example a membrane, a membrane slide or a microtiter plate can also be used as a calibration device, the invention is described in terms of a calibration slide. Given the fact that signal generated from RLS particles does not fade or photobleach, such a calibration slide can be rendered permanent by coating the slide with an optically transmissive layer.

Examples of coating materials and their properties that are to be considered to be useful for the invention are given in Section 5.3 and Table 2 of Example 6.1. Other forms or formats for RLS detection instrumentation calibration can be developed by one skilled in the art depending upon the desired assay format to be read on the instrument and the instrument configuration (incident light source or light path, optics, filters, detector, and the like).

In another embodiment, the invention provides methods for preparing calibration devices comprising RLS particles. As described above, RLS particles are stable labels and that the light scattering signal is not subject to decay, bleaching or quenching. The method for preparing calibration devices comprise depositing predetermined quantities (or ratios of quantities) of RLS particles in or on a sample device and coating at least a portion of the sample device with a coating composition that forms an optically transmissive coat. Preferably different types, quantities or dilutions of RLS particles are deposited at a plurality of spatially discrete sites and/or spatially addressable sites in or on the sample device.

Accordingly, the calibration device of the invention comprises different amounts of light scattering label particles present at different sites on the device, and that the device is at least partially coated with an optically transmissive coating. In specific embodiments, the calibration device has at least one dilution series of RLS particles, e.g., a series of 2-fold, 5-fold, and/or 10-fold dilutions; and/or has a plurality of different types of particles (e.g. different sizes and/or shapes). Preferably, the calibration device is packaged with a data sheet providing calibration data for the calibration device. Alternatively, such calibration data can be written or enclosed on the device itself by techniques known in the art such that the calibration data can be automatically read by an instrument and incorporated into an analysis and its records.

For example, array (including Microarray) calibration devices can be prepared by placing dilutions of RLS particles on an array. A variety of techniques can be used to distribute precise amounts of the RLS particles on different locations on the assay, including robotic pipetting or printing. The array is then coated with an optically transmissive layer or otherwise preserved. After preserving, this calibration slide can be used to adjust or calibrate the corresponding light scattering signals across different detection instrument units and/or across different experiments or determinations with the same instrument. The use of such reproducible calibration sample devices therefore allows more direct comparison of experimental results obtained in different laboratories, with a higher level of confidence.

A variety of different types RLS particles can be used, and a single calibration device can have one or more different types of particles. Mixtures of two or more different types of RLS particles (e.g., different sizes and/or shapes) in different known proportions in one location or site on the calibration device can also be used. Preferably, the type of RLS particles on a calibration sample device includes the particle type or types present on a sample device with which the calibration device is used. In a specific embodiment, the RLS particles used on a calibration device include generally spherical gold, silver, and combined gold and silver particles of 20, 40, 60, 80, 100, and 120 nm diameter.

In related embodiments, the invention includes methods for analyzing scattered light signals produced by a set of light scattering particles using a calibration device. In one embodiment, the method comprises measuring light signals from a first set of light scattering particles under defined conditions of illumination and detection; and measuring scattered light signals from a known amount of light scattering particles that is present on a calibration device under the same conditions. A comparison of the scattered light signals provide an estimate of the amount of light scattering particles in the first set. This method can be used to calibrate and standardize instruments and reagents used in an experiment.

A calibration device can also provide reliable comparison of scattered light signals among two or more different experiments. The two or more different experiments can be performed under different experimental conditions, e.g., on the same or different apparatuses, at the same or different times. The light signals from each of the different experiments can be related to each other by a conversion factor, such as a scaling constant, a curve, an equation or a mathematical model. The conversion factor for each of the different experiments is provided by comparing the light signals from each experiment to light signals generated by known amounts of light scattering particles present on a calibration device under the respective experimental conditions. The process of normalizing the assay results then further comprises applying the conversion factor to the assay results. After application of the calibration standard and the conversion factor, the normalized assay results from the different experiments can then be directly compared, as the normalization process removes instrument-dependent factors that may affect the results. In a specific embodiment, the normalized assay results from the two or more different experiments are compared. In a specific embodiment, the same calibration device is used in the two or more different experiments.

In an exemplary embodiment, a calibration device is used to construct a standard curve based on at least one particle dilution series on one or more calibration devices for an analyte assay using RLS particle-labeled analyte. Either the same or different calibration device is used in various experiments, with the same or a different analyzer, in the same or a different laboratory. Where different calibration devices are used, the different devices have a calibration factor associated with the device that allows comparison with the other calibration sample device or devices. In a specific embodiment, the calibration devices are calibrated to a master calibration standard.

In an embodiment of the present invention, to produce a calibration slide, different or the same types of RLS particles are prepared in a spotting solution comprising polyvinyl alcohol (PVA) and dimethylsulfoxide (DMSO) at various concentrations as additives, preferably 12% DMSO and 5% PVOH w/v, although, a range of concentrations around this point are also viable, and also other formulations of these or similar additives known in the microarray art may also be used. The preferred formulation achieved RLS particle confluence, identical spot size independent of RLS particle concentration and identical feature morphology over all printed features. Assessment of feature intensities were accomplished by both manual methods and by instrument/software methods. Such calibrations provided a reproducible tool for instrument calibration, in particular, determination of dynamic range and lower limits of detection. The particles are spotted as a dilution series, generally ranging from about 100 optical density units to about 0.006 optical density units, which corresponds to approximately 10 to 0.0006 particles/square micrometer on the slide surface depending on the particle size. Preferably, a particular formulation of spotting solution and conditions for spotting is selected that provides a substantially uniform particle distribution across the spots and prevents non-particle scattering effects during drying on the slide. Serial dilutions are then generated and diluted particles are deposited onto the slide surface. Deposition of RLS particles can be accomplished using any of a variety of spotter instruments known in the microarray field including but not limited to quill or blunt pen mechanical spotters, solenoid-based non-contact fluid deposition spotters, piezo-electric non-contact spotters and ink jet non-contact spotters. Once deposited on the surface, the spots containing the RLS particles at higher dilutions are viewed using a dark field microscope and the individual particles are counted. Through knowledge of the level of dilution and particle number at a given level of dilution, one can determine the RLS particle surface density with a high degree of accuracy. Once this is known, calibration slides can be generated by spotting a dilution series of spots containing known densities of RLS particles. Once spotted, the slides are dried (or cured) and preserved. In a specific embodiment, this is accomplished by dipping the slide into a coating composition and allowing the slide to cure or dry, although other methods including but not limited to spraying or vapor deposition can also be employed. Once the calibration slide so formed is dry, it can be stored, preferably in a protected, dust-free environment such as a microscope slide storage box. The longevity and performance of some preserving agents over time is enhanced by storage under dark or substantially dark conditions.

In an embodiment of the present invention, to effect the calibration process, the following steps are generally taken. First the image of the test slide is captured using instrumentation configured for efficient dark field illumination and detection with a CCD camera. A wide variety of detection systems can be employed with the present invention as described in U.S. Patent No. 6,214,560, Yguerabide et al., and U.S. Patent No. 6,180,415, to Schultz et al., United States Provisional Applications Serial Nos. 60/317,543 entitled "Apparatus for Analyte Assays" and 60/364,962 entitled "Multiplexed Assays Using Resonance Light Scattering Particles" (involving signal generation and detection system), and United States Nonprovisional Application Serial Nos. 08/953,713 entitled "Analyte Assay Using Particulate Labels," and 10/084,844 entitled "Methods for Providing Extended Dynamic Range in Analyte Assays." The integrated intensity of the features due to the presence of RLS particles on the test slide relative to the non-feature (i.e., background) scattering signal is quantified from the image using a wide variety of commercially available or customized image analysis software. For example, in the field of microarrays, one can obtain such image analysis software commercially from, for example, Biodiscovery InC., located in Los Angeles, CA, USA, which offers a software package called Imagene; Imaging Research Inc. located in St. Catherines, Ontario, Canada, which offers a software package called Array Vision; Axon Instruments Inc., located in Union City, CA, USA, which offers a software package called GenePix, etc. An image of the calibration slide is similarly collected, or a previously collected image is used if the measurement conditions can be reliably reproduced on the instrument. The integrated intensity from the features on the calibration slide is similarly obtained though image analysis. The background-corrected integrated intensity from the calibration slide is correlated with the known particle surface densities on the calibration slide. The RLS particle signal from the test slide is correlated with the signal from the calibration slide to yield the RLS particle density of the test slide. In this way, for a given set of instrument parameters such as incident light bulb intensity, filters, exposure time, camera resolution and the like, one obtains correlated values for expected signal to background measurements that are directly correlated with the known surface density or concentration of RLS particle labels. This enables researchers in different locations with different instrument models or different units of the same model to calibrate their instruments relative to one another or relative to a universally accepted or mutually agreed groups of settings or parameters. This allows users to obtain more consistent results that can be correlated and interpreted with other experimental results produced at different times and/or by other users.

## 6. <u>EXAMPLES</u>

### 6.1. <u>EVALUATION OF COATING MATERIALS</u>

A number of candidate coating materials were tested. Several suitable coating materials have been identified, which fulfill the criteria of ease of application (sheeting, viscosity), dry time, refractive index, optical clarity, hardness/ scratch resistance, stability of raw material, solvent compatibility, and cost.

Tests were performed with microarray slides printed with a solution containing bare gold 80 nm particles using an automated microarray printing system (Cartesian Technologies, Irvine, California) and quill pens (Telechem International, Inc., Sunnyvale, California). A complete description of microarray technology including printing, slide processing and fluorescent detection can be found in Microarray Biochip Technology, Ed. Mark Schena, Eaton Publishing, Natick, MA., 2000. The pattern printed was of 5 replicates (row)/metacolumn. The particles were diluted from 50 optical density units (O.D.) by ½ over ½ - 2 fold dilutions over 8 steps (columns), such that the first sample concentration is 50 OD, the second sample is 50/2=25 OD, the third sample is 25/2=12.5 OD, and so on until 8 dilutions have been made with the 8th sample at 0.39 OD. Two Metarows containing 4 metacolumns each were printed on the slides. The slides were then treated with several washes in biological buffers containing one or all of the following. The combination of steps recreate the typical steps of prehybridization, hybridization and post-washing with the exceptions noted. In this example, no overnight hybridization was implemented and no cDNA was added to the hybridization.

3x or less SSC (sodium citrate)
0.1% w/v SDS (sodium dodecylsulfate)
0.2% w/v BSA (bovine serum albumin)
Casein
10mM PBS (phosphate buffered saline)
Purified Water
Forced Nitrogen Air for Drying

These conditions were adopted to approximate microarray manipulation during experimental processing. The actual conditions used in most experimental processes may be more rigorous and may deposit higher levels of scattering impurities on a processed microarray slide.

Images ofmicroarray features before and after coating with candidate coating materials were processed identically using a commercially available microarray image analysis program (Array Vision, Imaging Research, Inc., Ontario, Canada). Thus, the display ranges were matched, in addition to scanning exposure times on the instrument taking the measurement. All image data were collected using either the ArrayWorXs automated microarray processing system (Applied Precision, Issaquah, Washington) or the GSD-501 system (Genicon Sciences Corporation, San Diego, California).

Exemplary candidate coating materials tested included:

Fcll = FICOLL™ 50%
Kryln = KRYLON™ Clear Coat Acrylic
Rstlm = RUSTOLEUM™ Clear Coat Paint
PolyU = Polyurethane
PR = Combination (1:1) Plastic (CRAPTICS™) and RUSTOLEUM™ Clear Coat Paint
PVA = PolyVinylAlcohol - viscoelastic polymer, Celanese grade 203s, 205s.

All slides were coated by dipping, with drying/curing at standard temperature and pressure. Scans were taken before and after coating the slides. Scans taken before coating were of the slides in the cleanest state that can be achieved after spotting (i.e, there was no further processing of the slides after spotting). It should be noted that treatment with biological buffers has typically shown a significant introduction of background noise as a result of trapped salts, proteins, small molecules and particle contaminants. The preferred embodiment comprises PVA.

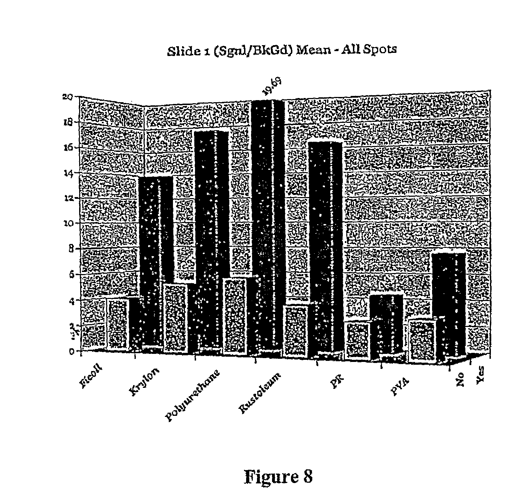

No loss in signal is observed, but a significant increase is observed in signal to background averages across all microarray features. Figure 8 is a graph showing representative average signal to noise ratios for exemplary coatings. As shown, there was a dramatic increase in signal to background averages across all spots on "Preserved" slides. In some of the better performing coatings, signal to background averages increase approximately 4-fold relative to uncoated slides.

Described hereinbelow in Table 2 are the results of some of the candidate coating materials tested. The Tyndall effect is the scattering of visible light in all directions, and a Tyndall Beam is used which measures the light scattering properties of the coating materials. The color of the coating material is given when the coating is just applied (wet), and after it has cured to form a coating (dry).

Table 2

| Coating Materials | Original Purpose | Source | Distributed Form | Tyndall Liquid/ Slide | Color Wet/Dry | Finish | Comments |
|---|---|---|---|---|---|---|---|
| Parks Clear Lacquer | Protective Wood Finish | Parks | Liquid - Petroleum | Scatters/ Scatters | Straw/ Straw | High Gloss | Mixed Organic base; Transparifies Slide Debris, Residue and Scratches; Straw Yellow Hue |
| Deft Lacquer | Protective Wood Finish | Deft | Liquid - Petroleum | Scatters/ Scatters | Straw/ Straw | High Gloss | Good Clarity with Straw Yellow Hue; Easily Applied; Xylene/ Aliphatic Base Transparifies Slide Debris, Residue & Scratches |

(continued)

| Coating Materials | Original Purpose | Source | Distributed Form | Tyndall Liquid/ Slide | Color Wet/Dry | Finish | Comments |
|---|---|---|---|---|---|---|---|
| Ficoll/ Poly-sucrose | Sucrose Polymer | Sigma/ Aldrich; Pharmacia | Powder | No Scatter/No Scatter | Clear/ Clear | High Gloss | Excellent coating on some surfaces; Poor wetting on hydrophobic surfaces |
| Zar Fast Dry Poly-Urethane | Protective Wood Finish | Zar (UGL) | Liquid - Petroleum | Scatters/ Scatters | Straw/ Straw | High Gloss | Good Clarity with Straw Yellow Hue; easily applied; mineral spirit base. Tranfparifies slide debris, residue and scratches |
| Zar Interior Grade Poly-Urethane | Protective Wood Finish | Zar (UGL) | Liquid - Petroleum | Scatters/ Scatters | Straw/ Straw | High Gloss | Good Clarity with Straw Yellow Hue; easily applied; mineral spirit base. Tranfparifies slide debris, residue and scratches |
| PolyVinyl Alcohol | Vinyl Viscoelastic Polymer | Sigma | Powder | No Scatter/No Scatter | Clear/ Clear | High Gloss | Good Clarity; Some particulate; Weak Scatter; Easily Applied; Transparifies Slide Debris, Residue & Scratches |
| Rustoleum Lacquer | Protective Clear Coat | Rustoleum | Aerosol - Petroleum | Scatters/ Scatters | Clear/ Clear | High Gloss | Excellent Clarity; Low Level Scatter; Easily Applied; Transparifies Slide Debris, Residue & Scratches |

(continued)

| Coating Materials | Original Purpose | Source | Distributed Form | Tyndall Liquid/ Slide | Color Wet/Dry | Finish | Comments |
|---|---|---|---|---|---|---|---|
| Varathane 900 Poly-Urethane | Protective Wood Finish | Varathane | Liquid - Petroleum | Scatters/ Scatters | Amber/ Amber | High Gloss | Good Clarity Despite Amber Hue; Easily Applied; Oil Base Transparifies Slide Debris, Residue & Scratches |
| Beta Pinene | Viscoelastic Polymer | Sigma | Crystals/ Pellets | No Scatter/No Scatter | Amber/ Amber | High Gloss | Excellent Clarity. Candidate organic solvents include Toluene |

## 6.2. TRANSPARIFYING AND PRESERVING MEMBRANES

This example describes the production of nitrocellulose membrane bound glass slides for use with RLS particles. Further described is a process of nitrocellulose membrane transparification and preserving, using a solution that both clarifies the membrane and solidifies to protect the membrane. Candidates for membrane transparification and preserving solutions were identified, which fulfill the criteria of ease of application (sheeting, viscosity), dry time, refractive index, optical clarity, hardness/scratch resistance of polymer, stability of raw material, solvent compatibility, and cost.

1. Production of Nitrocellulose Membranes

Materials:

Corning Gold Seal Slides (any plain glass slide may be used)

3M Optical Adhesives 8141, 8142, 8161 or 9483 (any of those listed can be used)
Pall Nitrocellulose Membrane (although any manufacturer's membrane may be substituted)
Rigid Tube Approximately ½ inch in Diameter (used to apply pressure to the adhesive)
Razor Blade
Tape

Process:

A panel of slides was arranged in a rectangle composed of 7 columns and 2 rows. The slides were immobilized with standard lab tape to eliminate the possibility of movement during application of the adhesive and membrane. A segment of 2-sided optical adhesive was cut to match the rectangular panel and applied by first affixing one edge to the laboratory bench proximal and squared off with the rectangular grid such that release of tension would result in the adhesive flap dropping squarely on the slides. Tension is maintained with one hand as the other applies pressure to the contact edge of the adhesive with the rigid tube being applied by the opposite hand. The contact edge of the adhesive is moved forward by continued application of firm and even pressure across the tube's length as the opposite hand slowly releases pressure. The description given here is the manual manifestation of Nip Roll Lamination - a process fully characterized and familiar in industrial settings. The result is the bubble free application of an optically clear adhesive on which nitrocellulose membrane is applied.

The application of the nitrocellulose only requires a proper fit and gentle pressure smoothly applied across the surface with a hand or roller so to ensure proper adhesion. The panel of slides is then finished by segmentation with a razor blade to minimize any rough edges.

Other methods for preparing a membrane substrate or a solid support that are known in the art, such as fluid or spin casting polymer matrices onto surfaces, can also be used with the present invention.

2. Microarray Layout, Membrane Transparification and Preservation

Materials:

| | |
|---|---|
| Deft Clear Lacquer | Cartesian Technologies Arrayer |
| Parks Clear Lacquer | RLS-view Instrument |
| 2-Butoxyethanol | |

Array Pattern:

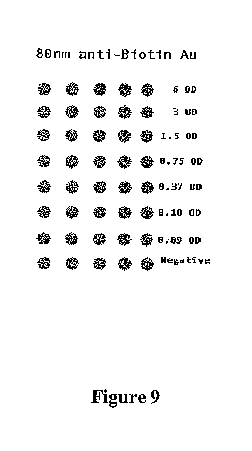

Nitrocellulose membranes as prepared above were spotted on a Cartesian Technologies arrayer in a rectangular array pattern as shown in Figure 9 with 80nm anti-biotin bound gold RLS particles.

Spotting was done in a formulation of 150mM NaCl and 5% Bovine Serum Albumin. After arraying the slides were washed with distilled water. The highest and lowest concentrations of anti-biotin 80nm Gold spotted were 6 OD and 0.09 OD respectively. These concentrations are quite low relative to what is achievable in a bioassay. The background levels observed on a 20 second scan on the RLS-view instrument (Genicon Sciences, San Diego, CA) were approximately 75 counts/sec for the best exemplary membrane preserving candidate. All membrane slides were dip coated and cured at standard temperature and pressure.

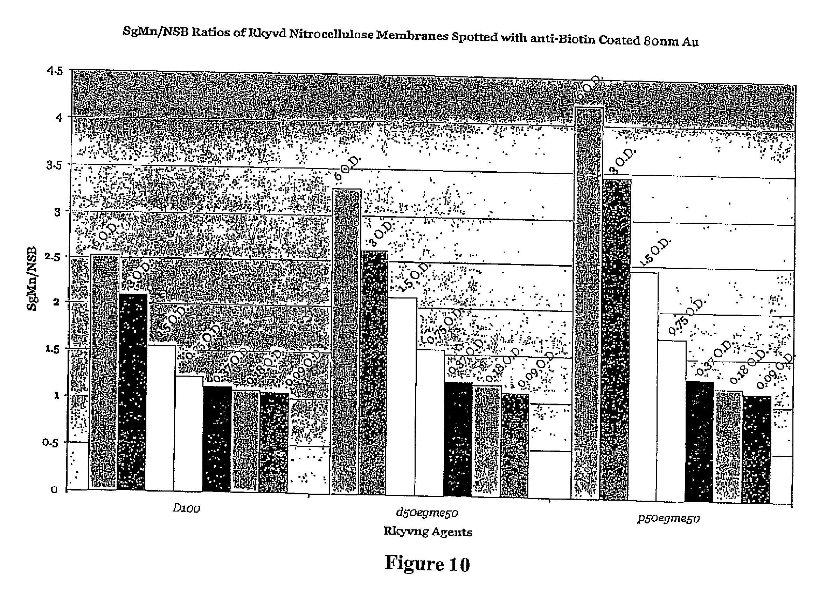

Described in Table 3 are the abbreviations used in the experiments and Figure 10. They represent common usages and three transparifying/preservation candidates chosen for this experiment.

**Table 3**

| Abbreviation | Definition | Refractive Index |
|---|---|---|
| Rkyv, Rkyvd, or, Rkyvng | Experimental usage of Preserve, Preserved, and Preserving | Not Applicable |
| Au | Gold | Not Applicable |
| D100, or, d100 | Deft Clear Lacquer 100% volume | 1.436 (liquid) |
| D50 EGME50, or, d50egme50 | Deft Lacquer 50% 2-Butoxyethanol 50% (v/v) | 1.427 (liquid) |
| P50 EGME50, or, p50egme50 | Parks Lacquer 50% 2-Butoxyethanol 50% (v/v) | 1.422 (liquid) |

TIF images of transparified membrane slides were captured on an RLS-view instrument. The spotting scheme is as indicated in Figure 9. The arrays were applied in triplicate with duplicate slides prepared with each of the transparifying/preservation candidates. Each of the images was held to the same screen stretch and instrument exposure time (20 seconds).

The Parks Clear Lacquer prepared with 50% v/v 2-Butoxyethanol shows indications of lower background and particulate inclusion; however, a second interesting point was observed. The spot intensities observed on the arrays coated with Deft Clear Lacquer 100% appear to be greater than the spots with other two coatings, attributable to the greater refractive index, or, thicker tegument produced by the undiluted Deft Lacquer.

Figure 10 is a graph showing signal to non-specific background ratios for 3 coating materials on nitrocellulose membranes. The calculations were arrived at by dividing the signal mean of the spots observed by the average of negative spots in each array. This result is represented as the Average SgMn/NSB (Average Signal Mean divided by Non-specific Background). The first and last bars in each set, corresponding to Rows 1 and 7 of Figure 9, represent the highest and lowest anti-biotin 80nm gold RLS particle densities, respectively. Row 8 is excluded as it is taken into account in the calculations (see Table 3 for the abbreviation definitions). As indicated above, this example illustrates an effective one-step transparifying and preserving method. Additionally, a class of coating compositions is identified, for which candidate coating materials are readily available, inexpensive and easily modified in favor of more desirable properties (e.g., refractive index increase, viscosity reduction, volatility, etc.). These coating compositions and methods are an extension of what is described in Example 1, with the exception that cellulose nitrate membranes are made transparent during the method.

One skilled in the art will readily appreciate that the present invention is well adapted to carry out the objects and obtain the ends and advantages mentioned, as well as those inherent therein. The methods, variances, and compositions described herein as presently representative of preferred embodiments are exemplary and are not intended as limitations on the scope of the invention. Changes therein and other uses will occur to those skilled in the art, which are encompassed within the spirit of the invention, are defined by the scope of the claims.

It will be readily apparent to one skilled in the art that varying substitutions and modifications may be made to the invention disclosed herein without departing from the scope and spirit of the invention. For example, using other sample devices and/or labeling techniques are all within the scope of the present invention. Thus, such additional embodiments are within the scope of the present invention and the following claims.

The invention illustratively described herein suitably may be practiced in the absence of any element or elements, limitation or limitations which is not specifically disclosed herein and/or may suitably be practiced in the presence of an additional element or elements, limitation or limitations. Thus, for example, in each instance herein any of the terms "comprising", "consisting essentially of" and "consisting of" may be replaced with either of the other two terms for other embodiments. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

In addition, where features or aspects of the invention are described in terms of Markush groups or other grouping of alternatives, those skilled in the art will recognize that the invention is also thereby described in terms of any individual member or subgroup of members of the Markush group or other group.

Where a component or limitation is described with a variety of different possible numbers or dimensions associated with that component or limitation, in additional embodiments, the component or limitation is in a range specified by taking any two of the particular values provided as the endpoints of the range. The range includes the endpoints unless clearly indicated to the contrary.

Thus, additional embodiments are within the scope of the invention and within the following claims.

**Claims**

1. A method for preserving a sample comprising light scattering particles or having been contacted with light scattering particles, the method comprising applying a coating composition to at least a portion of the sample to form an optically transmissive coating, wherein the light scattering particles are of a size between 1 and 500 nm inclusive, and wherein light scattered from one or more said particles can be detected by a human eye with less than 500 times magnification and without electronic amplification.

2. A method as claimed in claim 1, wherein said sample is present on a sample device, preferably wherein the sample device is selected from the group consisting of a slide, an array chip, a microtiter plate, a microarray, a membrane, a glass substrate and a film.

3. A method as claimed in claim 1 or claim 2, wherein said coating comprises:

   (a) at least one polymeric compound selected from the group consisting of alkyd resins, acrylics, carbohydrate polymers, epoxy resins, polyesters, polyurethanes, polyvinyl alcohols, polyvinyl acetates, terpenes, urethane alkyds, and urethane oils; or
   (b) a lacquer, a varnish or a wood finishing lacquer.

4. A method as claimed in any preceding claim, wherein said coating composition is prepared by a method comprising adding a diluent to a coating composition comprising a polymeric compound, preferably wherein said coating composition comprises a wood finishing lacquer and the diluent is 2-butanone, 2-butoxyethanol, methyl ethyl ketone, ethylene glycol monobutyl ether or a combination thereof.

5. The method as claimed in any preceding claim, further comprising, after the applying step,

   (a) curing the coating composition such that the coating becomes permanent or solid; or
   (b) storing the sample under dark conditions; or

(c) removing at least a portion of a coating formed by a previous step and applying to the sample another coating composition to form another optically transmissive coating.

6. A method as claimed in any preceding claim, further comprising, after the applying step, the steps of storing the sample and detecting light scattered from the light scattering particles on the sample, wherein the detecting occurs following storing the sample for a period of one day, one week, one month, six months or one year, preferably wherein the storing and detecting steps are performed a plurality of times.

7. A method as claimed in any preceding claim, wherein the sample is present on a membrane, and the coating composition modifies the membrane such that less light is scattered by the membrane, preferably wherein the coating composition comprises:

    (a) one or more terpenes; or
    (b) betapinene, and either xylene, toluene, or both; or
    (c) wood finishing lacquer; or
    (d) a wood finishing lacquer and 2-butanone, 2-butoxyethanol, methyl ethyl ketone or ethylene glycol monobutyl ether or a combination thereof.

8. A method as claimed in any of claims 2 to 7, wherein the membrane:

    (a) is made of cellulose nitrate, nylon, cellulose or polyvinylidene fluoride; and/or
    (b) is attached to or supported by a frame; and/or
    (c) is associated with an optically transmissive solid phase; preferably

wherein the solid phase is glass or plastic.

9. A method for reducing background light scattering or enhancing specific detection of light scattering particle labels in a sample comprising light scattering particles or having been contacted with light scattering particles, the method comprising,
coating at least a portion of said sample with a coating composition, wherein the coating composition forms an optically transmissive coating, and wherein the refractive index of the optically transmissive coating provides reduced background light scattering and/or refractive index enhancement for detection of light scattered from the labels.

10. A method as claimed in claim 9, wherein the sample is on a sample device selected from the group consisting of a solid phase array, a slide, an array chip, a microtiter plate, a membrane.

11. A method as claimed in claim 9 or claim 10, wherein light scattered from the labels is detected prior to storage of the sample and is an indication of the presence or amount or both of at least one analyte on said sample device.

12. A method for preparing a calibration device, comprising:

    depositing a known amount of light scattering particles at one or more discrete locations on a sample device; and coating the sample device with a coating composition that forms an optically transmissive coating, wherein the light scattering particles are of a size between 1 and 500 nm inclusive, and wherein light scattered from one or more said particles can be detected by a human eye with less than 500 times magnification and without electronic amplification.

13. A method as claimed in claim 12, wherein the calibration device is selected from the group consisting of an array, a chip, a slide, and a plate.

14. A method as claimed in claim 12 or claim 13,, further comprising calibrating the calibration device to a master calibration standard.

15. A method for analyzing light signals generated by a set of light scattering particles comprising:

    (a) measuring scattered light signals from a set of light scattering particles under defined conditions;
    (b) measuring scattered light signals from a known amount of light scattering particles under the same defined conditions; and

comparing the scattered light signals from steps (a) and (b) to provide an estimate of the amount of light scattering particles in the set in step (a), wherein said known amount of light scattering particles present on a calibration device is preserved permanently with an optically transmissive coating.

16. A method as claimed in any preceding claim, wherein said light scattering particles are gold or silver particles, and said light scattering particles have a diameter selecting from the group consisting of 20,40,60,80,100,120,140 and 200 nm.

17. A sample device comprising at least one optically transmissive coating that is formed on a sample that comprises light scattering particles or that has been contacted with light scattering particles, wherein the light scattering particles are of a size between 1 and 500 nm inclusive, and wherein the light scattered from one or more the particles can be detected by a human eye with less than 500 times magnification and without electronic amplification.

18. A sample device as claimed in claim 17, wherein at least one of the optically transmissive coating comprises at least one polymeric compound selected from the group consisting of alkyd resins, acrylics, carbohydrate polymers, epoxy resins, polyesters, polyurethanes, polyvinyl alcohols, polyvinyl acetates, terpenes, urethane alkyds, and urethane oils.

19. A sample device as claimed in claim 17 or claim 18, wherein the sample device is

   (a) a solid phase array, a slide, a microtiter plate, an array chip, a microarray, a membrane, a glass substrate, a film; or
   (b) a forensic sample device, an identification sample device, or a clinical sample device.

20. A sample device as claimed in any of claims 17 to 19, wherein the light scattering particles are gold or silver particles, and the light scattering particles have a diameter selecting from the group consisting of 20,40,60,80,100,120,140 and 200 nm.

21. A calibration device comprising at least one discrete location that comprises a known amount of light scattering particles and that is preserved permanently with an optically transmissive coating, wherein the light scattering particles are of a size between 1 and 500 nm inclusive, and wherein light scattered from one or more said particles can be detected by a human eye with less than 500 times magnification and without electronic amplification.

22. A calibration device as claimed in claim 21, wherein the light scattering particles:

   (a) are present in the location at a surface density of from 10 to 0.0006 particles/square micrometer; and/or
   (b) are gold or silver particles, and the light scattering particles have a diameter selecting from the group consisting of 20,40,60,80,100,120,140 and 200 nm.

23. A kit comprising:

   a coating composition; and
   a set of instructions for coating a sample comprising light scattering particles or having been contacted with light scattering particles with
   said coating composition.

24. A kit as claimed in claim 23, further comprising:

   (a) at least one or more of a curing agent, a removal agent, a diluent or light scattering particles; or
   (b) at least one sample device; or
   (c) an instrument for detection of light scattering particles.

25. A kit as claimed in claim 23 or claim 24, wherein the light scattering particle comprise moieties that bind to analytes under binding conditions.

Figure 1A

Figure 1B

Figure 1C

Figure 2

Figure 3A

Figure 3B

Figure 4A

Figure 4B

**Figure 5A**

**Figure 5B**

**Figure 5C**

Figure 6A

Figure 6B

Figure 6C

# PARTICLE TYPE CONFIGURATION

**PARTICLE COMPOSITION**

- METAL-LIKE OR OTHER-
- MIXED COMPOSITION
- SURFACE COATING BY-
- BINDING AGENT(S)
- ACTIVATED POLYMER
- POLYMER
- MIXED SURFACE COATING
- OTHER

**PARTICLE SHAPE**

- SPHERICAL
- OVAL / ELLIPSODIAL
- ASYMMETRICAL
- MULTI-PARTICLE-
  AGGREGATES

**PARTICLE SIZE**

- VERY SMALL 1- 10 NM
- SMALL 11-40 NM
- MEDIUM 41-100 NM
- LARGE 100-250 NM
- VERY LARGE > 250 NM

**PARTICLE HOMOGENEITY**

- SIZE DISTRIBUTION
- SHAPE DISTRIBUTION

SELECTION OF VARIOUS ASPECTS OF THE ABOVE BOXES DETERMINES
WHAT TYPES OF LIGHT SCATTERING PROPERTIES ARE DETECTABLE. THE
ABOVE PROCESS LEADS TO SPECIFIC TEST KITS.

Figure 7

EP 2 051 074 A1

Slide 1 (Sgnl/BkGd) Mean - All Spots

Figure 8

Figure 9

SgMn/NSB Ratios of Rkyvd Nitrocellulose Membranes Spotted with anti-Biotin Coated 8onm Au

Figure 10

**Europäisches Patentamt**
**European Patent Office**
**Office européen des brevets**

## EUROPEAN SEARCH REPORT

Application Number

EP 09 15 2328

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 98/37417 A (THE REGENTS OF THE UNIVERSITY OF CALIFORNIA) 27 August 1998 (1998-08-27) * page 5, line 32 - line 33 * * page 9, line 32 - line 33 * * page 11, line 31 * * page 42, line 31 - page 44, line 2 * * page 67; claim 40 * * page 21, line 16 - line 25 * ----- | 1-11, 17-20 | INV. G01N33/53 G01N33/543 G01N33/557 G01N1/14 |
| X | US 4 853 262 A (HORIE ET AL) 1 August 1989 (1989-08-01) * column 2, line 29 - line 68 * ----- | 23-25 | |
| X | CUNHA G R ET AL: "Identification in histological sections of species origin of cells from mouse, rat and human" STAIN TECHNOLOGY 1984 UNITED STATES, vol. 59, no. 1, 1984, pages 7-12, XP008056212 * page 7 * ----- | 23-25 | |
| X | EP 0 259 137 A (CARIBBEAN MICROPARTICLES CORPORATION) 9 March 1988 (1988-03-09) * page 2, line 52 * ----- | 23-25 | TECHNICAL FIELDS SEARCHED (IPC) G01N |
| X | GB 2 161 822 A (FARMITALIA CARLO * ERBA S P A) 22 January 1986 (1986-01-22) * page 1, line 40 - line 43 * ----- | 23-25 | |
| D,Y | US 6 214 560 B1 (YGUERABIDE JUAN ET AL) 10 April 2001 (2001-04-10) * column 112; claim 1; example 22 * * columns 45-47,58 * ----- | 1-25 | |

-/--

The present search report has been drawn up for all claims

| Place of search | Date of completion of the search | Examiner |
|---|---|---|
| The Hague | 3 March 2009 | Lunter, Pim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 09 15 2328

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RAMIREZ P ET AL: "ELECTROPHORETIC TRANSFER OF VIRAL PROTEINS TO NITRO CELLULOSE SHEETS AND DETECTION WITH PEROXIDASE BOUND LECTINS AND PROTEIN A" JOURNAL OF IMMUNOLOGICAL METHODS, vol. 62, no. 1, 1983, pages 15-22, XP002355894 ISSN: 0022-1759 * page 17 - page 18 * | 1-25 | |
| X | ANONYMOUS: "Polyvinyl alcohol" INTERNET ARTICLE, [Online] 13 December 2000 (2000-12-13), XP002357096 Retrieved from the Internet: URL:web.archive.org/web/20001213052600//http://www.chemistrystore.com/Polyvinyl_alcohol.htm> [retrieved on 2005-12-08] * the whole document * | 23-25 | |
| X | LOVELAND R P ET AL: "MOUNTING MEDIA FOR MICROSCOPY" MICROSCOPE, CARSHALTON BEECHES, US, vol. 34, no. 3, 1 January 1986 (1986-01-01), pages 181-242, XP008079562 ISSN: 0026-282X | 23-25 | TECHNICAL FIELDS SEARCHED (IPC) |
| Y | * the whole document * * pages 183-184 * * pages 211-212 * | 1-25 | |

The present search report has been drawn up for all claims

| Place of search | Date of completion of the search | Examiner |
|---|---|---|
| The Hague | 3 March 2009 | Lunter, Pim |

**ANNEX TO THE EUROPEAN SEARCH REPORT**
**ON EUROPEAN PATENT APPLICATION NO.**

EP 09 15 2328

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2009

| Patent document cited in search report | | Publication date | Patent family member(s) | | | Publication date |
|---|---|---|---|---|---|---|
| WO 9837417 | A | 27-08-1998 | AT | 349697 | T | 15-01-2007 |
| | | | AU | 748939 | B2 | 13-06-2002 |
| | | | AU | 6169098 | A | 09-09-1998 |
| | | | CA | 2280794 | A1 | 27-08-1998 |
| | | | EP | 0979409 | A1 | 16-02-2000 |
| | | | JP | 4209471 | B2 | 14-01-2009 |
| | | | JP | 2001513198 | T | 28-08-2001 |
| US 4853262 | A | 01-08-1989 | JP | 1814392 | C | 18-01-1994 |
| | | | JP | 5019684 | B | 17-03-1993 |
| | | | JP | 62038408 | A | 19-02-1987 |
| EP 0259137 | A | 09-03-1988 | DE | 3770894 | D1 | 25-07-1991 |
| | | | US | 4689307 | A | 25-08-1987 |
| GB 2161822 | A | 22-01-1986 | IT | 1187754 | B | 23-12-1987 |
| US 6214560 | B1 | 10-04-2001 | US | 2009004757 | A1 | 01-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 09948058 B
- US 6214560 B, Yguerabide
- US 9706584 W
- WO 9740181 A
- US 9823160 W, Yguerabide
- WO 9920789 A
- US 95371397 A, Yguerabide
- US 08484402 A, Yguerabide
- US 9802995 W, Schultz
- WO 9837417 A

- US 6180415 B, Schultz
- US 740615 A, Schultz
- US 5350697 A, Swope
- US 4446238 A, De Mey
- US 4752567 A, DeBrabander
- US 6165798 A
- US 60317543 B
- US 60364962 B
- US 08953713 B
- US 10084844 B

**Non-patent literature cited in the description**

- **YGUERABIDE ; YGUERABIDE.** *Anal. Biochem.,* 1998, vol. 261, 157-176
- *Anal. Biochem.,* 1998, vol. 262, 137-156

- **SCHULTZ et al.** *Proc. Natl. Acad. Sci.,* 2000, vol. 97, 995-1001
- **DEBRABANDER et al.** *Cell Motility and the Cytoskeleton,* 1986, vol. 6, 105-113